# EUROPÄISCHE PATENTSCHRIFT

(11) **EP 2 354 121 B1**
(45) Veröffentlichungstag und Bekanntmachung des Hinweises auf die Patenterteilung: **13.02.2013**
(21) Anmeldenummer: 10152769.5
(22) Anmeldetag: 05.02.2010
(51) Int. Cl.: C07C 69/96, C07C 277/08, C07C 279/04

(54) **Hexaorganoguanidinium-organocarbonate, ihre Herstellung und ihre Verwendung**
Hexa-organic guanidinium organocarbonate, production and use of same
Carbonate organique à base d'hexa-organo-guanidine, sa fabrication et son utilisation

(43) Veröffentlichungstag der Anmeldung: 10.08.2011
(73) Patentinhaber: PHILIPPS-UNIVERSITÄT MARBURG, 35037 Marburg (DE)
(72) Erfinder: Sundermeyer, Jörg, 35041 Marburg (DE); Oelkers, Benjamin, 35039 Marburg (DE)
(74) Vertreter: Buchhold, Jürgen

(56) Entgegenhaltungen:
- WO-A1-2006/027069
- WO-A2-2008/052861
- US-A1- 2008 058 549
- B.ALBERT UND M.JANSEN: "Zur solvensfreien Darstellung von Tetramethylammoniumsalzen" ZEITSCHRIFT FÜR ANORGANISCHE UND ALLGEMEINE CHEMIE, Bd. 621, 1995, Seiten 1735-1740, XP009135345

## Beschreibung

Die vorliegende Erfindung beschreibt Hexaorganoguanidinium-organocarbonate, die aus Pentaorganoguanidinen und Diorganocarbonaten in einer 100% atomökonomischen Reaktion hergestellt werden. Die erfindungsgemäßen Hexaorganoguanidinium-organocarbonate sind ionische Flüssigkeiten. Durch anschließende Umsetzung mit Säuren HX kann das Carbonatanion durch beliebige Anionen X ausgetauscht werden, wodurch weitere Hexaorganoguanidinium-basierte ionische Flüssigkeiten zugänglich werden. Bei dieser Anion-Metathese kommt es zur Decarboxylierung des Organocarbonats. Deshalb wird das Reaktionsgleichgewicht der Metathese auf die Produktseite verschoben. Die beanspruchte Reaktionssequenz eignet sich zur Herstellung großer Mengen besonders reiner Guanidiniumsalze mit einem Schmelzpunkt von unter 100°C.

### Beschreibung und Einleitung des allgemeinen Gebietes der Erfindung

Die vorliegende Erfindung betrifft das Gebiet der organischen Chemie, insbesondere das Gebiet der ionischen Flüssigkeiten.

### Stand der Technik

lonische Flüssigkeiten besitzen zahlreiche Verwendungsmöglichkeiten, beispielsweise als technische Lösungsmittel oder Betriebsmittel, als Schmiermittel, in der Katalyse und als Elektrolyte in Batterien.

Gewünscht sind dabei möglichst einfache und kostengünstige Verfahren zu ihrer Herstellung, wobei diese Verfahren möglichst selektiv sein sollen und Nebenprodukte oder Verunreinigungen aus metathetischen lonenaustauschprozessen so weit wie möglich zu vermeiden sind.

Eine Übersicht über ionische Flüssigkeiten, die bei Raumtemperatur stabil sind, sowie Verfahren zu ihrer Herstellung und ihre Verwendung als Lösungsmittel und für die Katalyse bietet T Welton, "Room-Temperature lonic Liquids. Solvents for Synthesis and Catalysis", Chem Rev 1999, 99, 2071-2083.

G Barcelo, D Grenouillet, J-P Senet und G Sennyey beschreiben in "Pentaalkylguanidines as etherification and esterification catalysts", Tetrahedron 1990, 46, 1839-1848 die Eignung von Pentaalkylguanidinen als Katalysatoren für die Herstellung von Aryl- und Aralkylethern aus Organocarbonaten und für die Methylierung von Phenolen mit Dimethylcarbonat. Bei diesen Reaktionen kommt es nicht zur Methylierung der Pentaalkylguanidine durch Dimethylcarbonat.

In DJ Heldebrant, CR Yonker, PG Jessop, L Phan: "Organic Liquid CO2 capture agents with high gravimetric CO2 capacity" Energy Environ. Sci. 2008, 1, 487-493 ist die reversible Bindung von CO₂ an organische Alkohole und Amidin- oder Guanidinbasen beschrieben. In einer weiteren Veröffentlichung von DJ Heldebrant, CR Yonker, PG Jessop, L Phan: "CO2-binding organic liquids (CO2BOLs for postcombustion CO2 capture." Energy Procedia 2009, 1, 1187-1195, wird die Verwendung dieser organischen Flüssigkeiten für die Bindung von CO₂ nach Verbrennungsprozessen beschrieben. Die in beiden Veröffentlichungen beschriebenen Reaktionen erlauben jedoch keine Herstellung von Hexaorganoguanidiniumkationen, und die entstehenden Verbindungen sind thermisch nicht stabil.

Die WO 2007 / 053 630 A1 beschreibt ein Verfahren zur Herstellung ionischer Flüssigkeiten durch Anionenaustausch. Dabei werden Carboxylate von Kationen, die mindestens ein Stickstoffatom enthalten, durch Anionenmetathese mit einer Säure HX in die entsprechenden Salze aus Kation und Anion X überführt. Gezeigt werden ausschließlich Imidazoliumkationen, und es werden keine Organocarbonate, sondern Carboxylate offenbart.

Die WO 2006 / 027070 A1 beschreibt ein Verfahren zur Herstellung heterocyclischer quartärer Ammonium-Verbindungen und/oder Guanidinium-Verbindungen, wobei die entsprechenden heterocyclischen Carboxylate anschließend mit einer anorganischen oder organischen Protonensäure umgesetzt werden. Dabei kommt es zur Anionenmetathese. Carboxylate unterscheiden sich von den in dieser Offenlegungsschrift beanspruchten Organocarbonaten und Hydrogencarbonaten in der Natur des Anions wie auch in dem 100% atomökonomischen Zugangsweg allein der Organocarbonate.

Die WO 2006 / 027069 A1 beschreibt Verfahren zur Herstellung heterocyclischer quartärer Ammonium-Verbindungen und/oder Guanidinium-Verbindungen. Dabei werden heterocyclische quartäre Ammonium- und/oder Guanidinium-carboxylate mit einem oxidierenden Agens in Gegenwart eines Metalls aus der 8. bis 10. Gruppe des Periodensystems oxidiert.

Die WO 2008 / 052861 A2 beschreibt ein Verfahren zur Herstellung von 4-Carboxylat-freien 1,3-heteroaromatischer Organocarbonate. Bei den Heteroaromaten handelt es sich beispielsweise um Imidazol und Pyrimidin. Hierbei wird eine N-Alkylierung des Heteroaromaten beispielsweise mit Dimethylcarbonat (DMC) erzielt. Im ersten Schritt entsteht ein N-Methylimidazolium-methylcarbonat.

Nachteilig wirkt sich aus, dass es bei Abwesenheit von Methanol zu Folgereaktionen kommen kann, wie etwa die Carboxylierung des Imidazols in C-2, C4 und C-5 Position (J. D. Holbrey, W. M. Reichert, I. Tkatchenko, E. Bouajila, O. Walter, I. Tommasid and R. D. Rogers, Chem. Commun., 2003, 28-29 sowie M. Smiglak, J. D. Holbrey, S. T. Griffin, W. M. Reichert, R. P. Swatloski, A. R. Katritzky, H. Yang, D. Zhang, K. Kirichenko and R. D. Rogers, Green Chem., 2007, 9, 90-98). Während Verbindung 1 und 2 im folgenden Schema durch Umsetzung mit Säuren HX unter Decarboxylierung in neue Imidazoliumsalze übergegen, stellen die in 4- oder 5-Position carboxylierten Nebenprodukte Verunreinigungen dar, die nur sehr schwer durch Reaktion mit HX entfernbar sind.

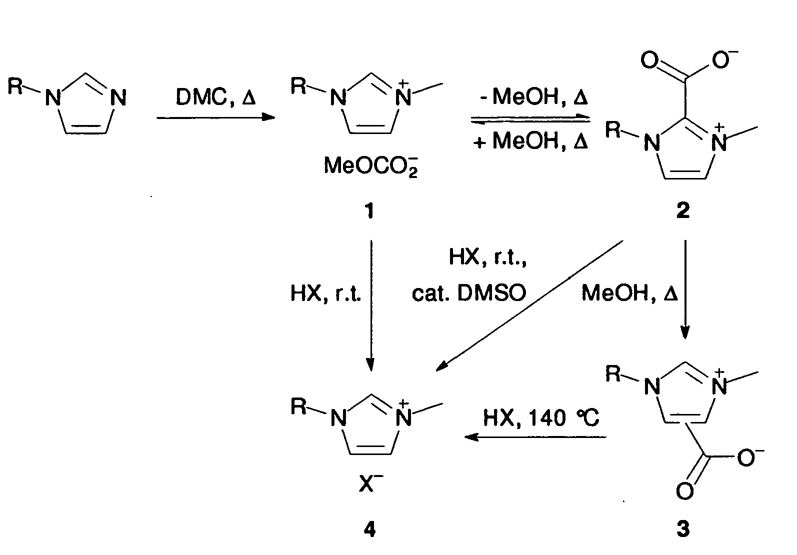

Die WO 2009 / 040242 A1 beschreibt ein Verfahren zur Herstellung von Imidazolium-organocarbonaten bei dem das Problem der Ringcarboxylierung durch Verwendung von Methanol und Arbeiten in einer aufwändigen Autoklavenreaktion unter erhöhten Drücken und Temperaturen weitgehend vermieden wird. Dennoch kann es je nach Reaktionsbedingungen neben einer (erwünschten) N-Alkylierung und Bildung eines Methylcarbonatanions auch zu einer (unerwünschten) Ring-Carboxylierung in 2-Stellung. kommen. In der Regel fällt ein Produktgemisch an. Im zweiten Verfahrensschritt wird das Imidazolium-organocarbonat in Methanol mit einer Säure oder einem sauren Salz umgesetzt. Bei dieser Metathese wird das Organocarbonatanion gegen das Anion der Säure oder des Salzes ausgetauscht.

Die letztgenannte Reaktion ermöglicht unter Freisetzung von Methanol und CO₂ die Herstellung ionischer Flüssigkeiten auf Basis von Imidazoliumkationen.

Der Stand der Technik kennt zahlreiche Möglichkeiten, ionische Flüssigkeiten mit Hexaorganoguanidinium-Kationen herzustellen (W. Kantlehner, E. Haug, W. W. Mergen, P. Speh, T. Maier, J. J. Kapassakalidis, H.-J. Bräuner and H. Hagen, Liebigs Ann. Chem., 1984, 108-126). Die bekannten Verfahren sind jedoch apparativ aufwändig, sie erfordern Phosgen-Technologie und das Arbeiten unter Schutzgas in wasserfreien Lösemitteln und führen zu unerwünschten Nebenprodukten. Die Überführung der bekannten Hexaorganoguanidinium-Halogenide, -Triflate und - Tosylate in andere Salze mittels Anionenmetathese ist eine unvorteilhafte Gleichgewichtsreaktion, die nicht zu den gewünschten Ausbeuten und Reinheiten der Anion-Metatheseprodukte führt. Zudem sind selbst Spuren anionischer Verunreinigungen wie Halogenid aus dem lonenaustausch-Gleichgewicht sehr unvorteilhaft, wenn es um Anwendungen der IL in elektrochemischen Zellen geht.

Die vorliegende Erfindung überwindet diese Nachteile, indem sie Hexaorganoguanidinium-organocarbonate, eine bislang nicht beschriebene Substanzklasse, bereitstellt. Vorstufe für diese neue Substanzklasse sind Pentaalkylguanidine, die im Rahmen der vorliegenden Erfindung nach literaturbekannten Vorschriften bzw. in Anlehnung daran hergestellt und vor der Verwendung im Feinvakuum destilliert wurden. Die Herstellung von Pentaalkylguanidinen ist beispielsweise in W. Kantlehner, E. Haug, W. W. Mergen, P. Speh, T. Maier, J. J. Kapassakalidis, H.-J. Bräuner und H. Hagen, Liebigs Ann. Chem. 1984, 108-126; G. Barcelo, D. Grenouillat, J.-P. Senet und G. Sennyey, Tetrahedron 1990, 46 (6), 1839-1848 beschrieben.
Die erfindungsgemäßen Hexaorganoguanidinium-organocarbonate lassen sich durch eine Anionenmetathese mittels Decarboxylierung quantitativ in weitere Hexaorganoguanidiniumsalze überführen.

### Aufgabe

Aufgabe der Erfindung ist es, neue Hexaorganoguanidinium-Salze sowie ein Verfahren zu ihrer Herstellung bereitzustellen.

### Lösung der Aufgabe

Die Aufgabe der Bereitstellung neuer Hexaorganoguanidinium-Salze wird erfindungsgemäß gelöst durch Verbindungen der Formel (I) 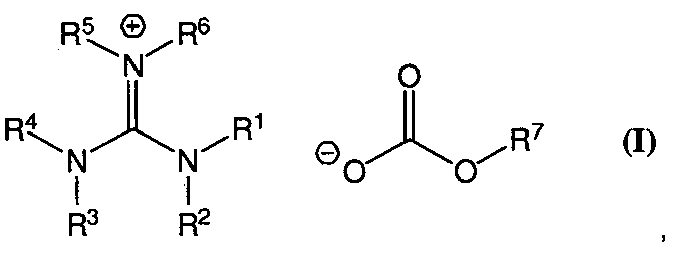 worin
R¹, R², R³, R⁴ und R⁵ unabhängig voneinander für eine lineare oder verzweigte acyclische oder cyclische Alkylgruppe mit 1 bis 20 Kohlenstoffatomen, eine Allyl- oder Vinylgruppe, eine Benzylgruppe, eine Aryl- oder Heteroarylgruppe stehen
oder
worin das Paar R¹ und R² sowie das Paar R³ und R⁴ unabhängig voneinander eine lineare oder verzweigte Alkylengruppe mit 2 bis 8 Kohlenstoffatomen darstellen, die jeweils mit einem Stickstoffatom des Guanidingerüstes eine cyclische sekundäre Aminogruppe -NR¹R² bzw. -NR³R⁴ bildet,
oder
worin das Paar R² und R³ eine lineare Alkylengruppe mit 2 bis 6 Kohlenstoffatomen darstellt, die mit dem zentralen Kohlenstoffatom und zwei Stickstoffatomen des Guanidingerüstes eine cyclische Gruppe ∼R ²-N-C-N-R³∼ bildet,
wobei in allen genannten Alkylengruppen eine -CH₂-Einheit optional durch O, NH oder NR¹ ersetzt sein kann, worin R¹ wie oben definiert ist, und worin cyclische sekundäre Aminogruppen -NR¹R² und -NR³R⁴ partiell ungesättigt sein können,
wobei im Falle des Vorliegens cyclischer Gruppen -NR¹R², -NR³R⁴ bzw. ∼ R²-N-C-N-R³∼ diejenigen Reste R¹ bis R⁴, welche nicht an den cyclischen Gruppen beteiligt sind, wie oben definiert sind,
und worin
R⁶ eine lineare oder verzweigte acyclische Alkylgruppe mit 1 bis 20 Kohlenstoffatomen, eine Benzyl- oder eine Allylgruppe ist,
und R⁷ eine lineare oder verzweigte acyclische oder cyclische Alkylgruppe mit 1 bis 20 Kohlenstoffatomen, eine Benzyl- oder eine Allylgruppe, eine Aryl- oder Heteroarylgruppe ist.

Die erfindungsgemäßen sechsfach an den drei Stickstoffatomen organosubstituierten Guanidinium-organocarbonate, das Verfahren zu ihrer Herstellung sowie die Verwendung dieser Guanidinium-organocarbonate sind nachfolgend erläutert, wobei die Erfindung alle nachfolgend aufgeführten Ausführungsformen einzeln und in Kombination miteinander umfasst.

Die Reste R¹ R², R³, R⁴ und R⁵ können unabhängig voneinander eine lineare oder verzweigte acyclische oder cyclische Alkylgruppe mit 1 bis 20 Kohlenstoffatomen, eine Allyl- oder Vinylgruppe, eine Benzyl-, Aryl- oder Heteroarylgruppe sein.

Im Rahmen der vorliegenden Erfindung werden die linearen oder verzweigten acyclischen Alkylgruppen mit 1 bis 20 Kohlenstoffatomen ausgewählt aus Methyl, Ethyl, n-Propyl, Isopropyl, 1-Butyl, 2-Butyl, tert.-Butyl, 1-Pentyl, 2-Pentyl, 3-Pentyl, 3-Methylbutyl, 2,2-Dimethylpropyl sowie allen Isomeren von Hexyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl und Eicosyl.

Dem Fachmann ist bekannt, dass cyclische Alkylgruppen mindestens drei Kohlenstoffatome enthalten müssen. Vorteilhaft umfassen cyclische Alkylgruppen im Rahmen der vorliegenden Erfindung Propyl-, Butyl-, Pentyl-, Hexyl-, Heptyl-, und Octyl-Ringe. Eine cyclische Alkylgruppe im Sinne der vorliegenden Erfindung wird ausgewählt aus den genannten ringförmigen Alkylgruppen, die keine weiteren Substituenten tragen, und aus den genannten ringförmigen Alkylgruppen, welche ihrerseits an eine oder mehrere acyclische Alkylgruppen gebunden sind. Im letztgenannten Fall kann die Bindung der cyclischen Alkylgruppe an ein Stickstoffatom des Guanidingerüstes gemäß Formel I über ein cyclisches oder ein acyclisches Kohlenstoffatom der cyclischen Alkylgruppe erfolgen. Cyclische Alkylgruppen enthalten gemäß obiger Definition des Begriffes "Alkylgruppe" ebenfalls insgesamt maximal 20 Kohlenstoffatome.

Handelt es sich bei einem der Reste R¹ ,R², R³, R⁴ und R⁵ um eine Arylgruppe, so ist diese ausgewählt aus Phenyl, Naphthyl und Anthracenyl.
Handelt es sich bei einem der Reste R¹, R², R³, R⁴ und R⁵ um eine Heteroarylgruppe, so ist diese ausgewählt aus Furanyl, Pyrrolyl, Thiophenyl, Imidazolyl, Pyrazolyl, Thiazolyl, Oxazolyl, Isooxazolyl, einem Oxadiazolyl, Pyridinyl, Pyrazinyl, Pyrimidinyl, Pyridazinyl, einem Triazinyl, einem Tetrazinyl, 1,4-Dioxinyl, einem Thiazinyl, einem Oxazinyl, einem Azepinyl, einem Diazepinyl, Benzofuranyl, Isobenzofuranyl, Indolyl, Isoindolyl, Benzothiophenyl, Benzo[c]thiophenyl, Benzimidazolyl, Purinyl, Indazolyl, Benzoxazolyl, Benzisoxazolyl, Benzothiazolyl, Chinolinyl, Isochinolinyl, Chinoxalinyl, Acridinyl, Chinazolinyl oder Cinnolinyl.

Handelt es sich bei einem der Reste R¹, R², R³, R⁴ und R⁵ um eine Allyl- oder Benzylgruppe, so kann diese optional durch eine oder mehrere Alkylgruppen mit je 1 bis 20 Kohlenstoffatomen oder eine Alkenylgruppe, insbesondere eine Vinyl-oder Allylgruppe, substituiert sein.

Die linearen oder verzweigten acyclischen oder cyclischen Alkylgruppen mit 1 bis 20 Kohlenstoffatomen sowie die Aryl-, Heteroaryl-, Allyl- und Benzylgruppen können auch noch weitere funktionelle Gruppen funktionalisierter Kohlenwasserstoffreste tragen. Beispielhaft, aber nicht erschöpfend, seien folgende funktionelle Gruppen genannt:
- CN, -F, -C1, -Br, -I, -SO₃⁻,
- primäre, sekundäre und tertiäre Aminogruppen -NH₂, -NHR^{a}, -NR^{a}R^{b},
- OR^{a}, -OH, -COR^{a}, -CHO,
- Si(OR^{a})(OR^{b})(OR^{c}), -Si(OR^{a})(OR^{b})R^{c}, -SiR^{a}R^{b}R^{c},
R^{a}, R^{b} und R^{c} stehen unabhängig voneinander für einen organischen Rest mit vorzugsweise 1 bis 20 Kohlenstoffatomen, besonders bevorzugt für einen Aryl-, Heteroaryl-, Allyl-, Benzyl-, Vinyl- oder Alkylrest und ganz besonders bevorzugt für einen aliphatischen Rest, insbesondere eine C1- bis C10-Alkylgruppe. Optional können die Reste R^{a}, R^{b}, R^{c} und R^{d} ganz oder teilweise fluoriert sein.

Enthalten die funktionellen Gruppen ihrerseits Kohlenstoffatome, beispielsweise im Falle von Ether-, Ester- oder Carbonylgruppen, so werden diese Kohlenstoffatome bei der Berechnung der Gesamtzahl von 1 bis 20 Kohlenstoffatomen der Alkylgruppen nicht berücksichtigt.

Alternativ können das Paar R¹ und R² sowie das Paar R³ und R⁴ unabhängig voneinander eine lineare oder verzweigte Alkylengruppe mit 2 bis 8 Kohlenstoffatomen darstellen, die jeweils mit einem Stickstoffatom des Guanidingerüstes eine cyclische sekundäre Aminogruppe -NR¹R² bzw. -NR³R⁴ bildet. Ebenso kann das Paar R² und R³ eine lineare oder verzweigte Alkylengruppe mit 2 bis 6 Kohlenstoffatomen darstellen, die mit dem zentralen Kohlenstoffatom und zwei Stickstoffatomen des Guanidingerüstes eine cyclische Gruppe ∼R²-N-C-N-R³∼ bildet. In allen genannten Alkylengruppen kann eine -CH₂-Einheit optional durch O, NH oder NR¹ ersetzt sein worin R¹ wie oben definiert ist.
Im Falle des Vorliegens cyclischer Gruppen -NR¹R², -NR³R⁴ bzw. ∼ R²-N-C-N-R³∼ sind diejenigen Reste R¹ bis R ⁴ welche nicht an den cyclischen Gruppen beteiligt sind, wie oben definiert.
Die Alkylengruppen können, wie oben ausgeführt, linear oder verzweigt sein. Vorteilhafte lineare Alkylengruppen sind beispielsweise die 1,2-Ethylen- und die 1,3-Propylengruppe. Eine vorteilhafte verzweigte Alkylengruppe ist die 1,2-Propylengruppe.

Die Alkylengruppen cyclischer Derivate können des Weiteren die oben bereits aufgeführten weiteren funktionelle Gruppen funktionalisierter Kohlenwasserstoffreste tragen. Besonders vorteilhaft tragen die Alkylengruppen NH-, Ester, CN-oder Carbonylgruppen.

Cyclische Gruppen -NR¹R² und -NR³R⁴ sind beispielsweise Piperidinylreste, Morpholinylreste, NH- oder NR¹-Piperazinylreste, Pyrrolidinylreste, Oxazolidinreste, NH- oder NR¹-Imidazolidinreste, Oxazolinylreste und Imidazolinylreste.

Beim Rest R⁶ handelt es sich um eine lineare oder verzweigte Alkylgruppe mit 1 bis 20 Kohlenstoffatomen, eine Benzyl- oder eine Allylgruppe. Dabei sind die linearen oder verzweigten acyclischen Alkylgruppen wie oben definiert. Alkyl-, Benzyl-und Allylgruppen R⁶ können optional substituiert sein, und zwar mit den oben für die Reste R¹ bis R⁵ beschriebenen weiteren Substituenten.
Beim Rest R⁷ handelt es sich um eine lineare oder verzweigte acyclische oder cyclische Alkylgruppe mit 1 bis 20 Kohlenstoffatomen, eine Benzyl- oder eine Allylgruppe, eine Aryl- oder eine Heteroarylgruppe. Dabei sind die linearen oder verzweigten acyclischen Alkylgruppen sowie die Aryl- und Heteroarylgruppen wie oben definiert.

Die erfindungsgemäßen Hexaorganoguanidinium-organocarbonate können symmetrisch oder unsymmetrisch sein. Symmetrisch sind dabei solche Verbindungen, bei denen es mindestens eine Dreh- oder Spiegelachse senkrecht zur Hauptebene des Hexaorganoguanidinium-Kations gibt. Dementsprechend werden Hexaorganoguanidinium-Kationen ohne eine Dreh- oder Spiegelachse senkrecht zur Hauptebene dieses Kations als unsymmetrisch bezeichnet.

In den nachfolgend beschriebenen Ausführungsformen der vorliegenden Erfindung sind solche Hexaorganoguandinium-organocarbonate bevorzugt, bei denen R⁶ eine Methyl-, Benzyl-, Allyl- oder Ethylgruppe ist. Ganz besonders bevorzugt ist R⁶ eine Methylgruppe.

In einer vorteilhaften Ausführungsform handelt es sich bei den Resten R¹ bis R⁵ unabhängig voneinander um lineare oder verzweigte acyclische Alkylgruppen.

Besonders bevorzugt handelt es sich bei den Resten R¹ bis R⁵ unabhängig voneinander um Methyl, Ethyl, Isopropyl, n-Propyl, n-Butyl und 2-Ethyl-hexyl (= Isooctyl).

In einer weiteren vorteilhaften Ausführungsform handelt es sich um Hexaorganoguanidinium-organocarbonate mit einer cyclischen sekundären Aminogruppe ∼ R²-N-C-N-R³∼. Die beiden Reste R¹ und R⁴ können dabei identisch oder voneinander verschieden sein.

In einer weiteren vorteilhaften Ausführungsform handelt es sich um Hexaorganoguanidinium-organocarbonate mit cyclischen sekundären Aminogruppen -NR¹R² und -NR³R⁴. Besonders vorteilhaft ist es, wenn diese beiden cyclischen Gruppen identisch sind.

Nachfolgend sind beispielhaft, aber nicht erschöpfend einige Beispiele erfindungsgemäßer um Hexaorganoguanidinium-organocarbonate mit cyclischen sekundären Aminogruppen aufgeführt:
1. Cyclische symmetrisch (R¹ = R⁴) und unsymmetrisch (R¹ ≠ R⁴) substituierte Abkömmlinge der sehr leicht und billig zugänglichen cyclischen Pentaalkylguanidine: 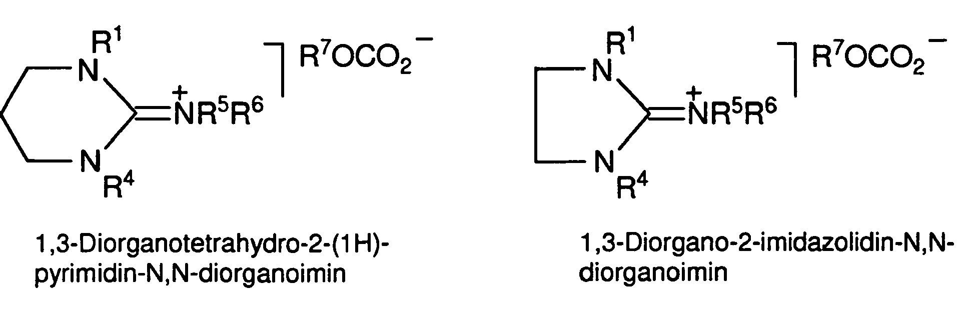 und auch verzweigte Alkylengruppen, z.B. Propanl,2-diyl: 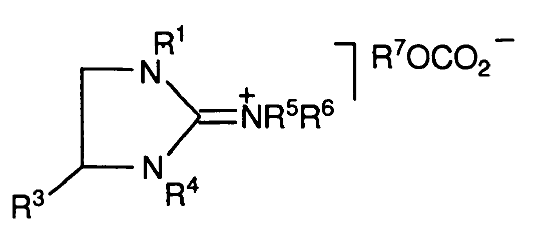 "Symmetrisch" bzw. "unsymmetrisch" bezieht sich hier auf das Substituentenmuster der zugrundeliegenden Pentaalkylguanidine.
2. Abkömmlinge von Pentaalkylguanidinen mit zyklischen sekundären Aminresten.
   Nachfolgend sind zwei Strukturen aufgeführt, die beispielhaft, aber nicht erschöpfend alle denkbaren Zyklen beschreiben: 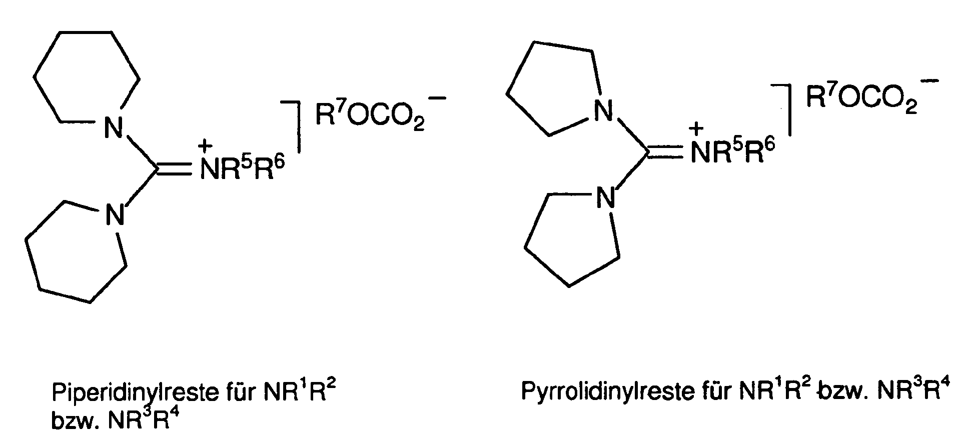
3. Analoges gilt für Alkylengruppen, bei denen eine -CH₂-Einheit durch O, NH oder NR¹ ersetzt ist: 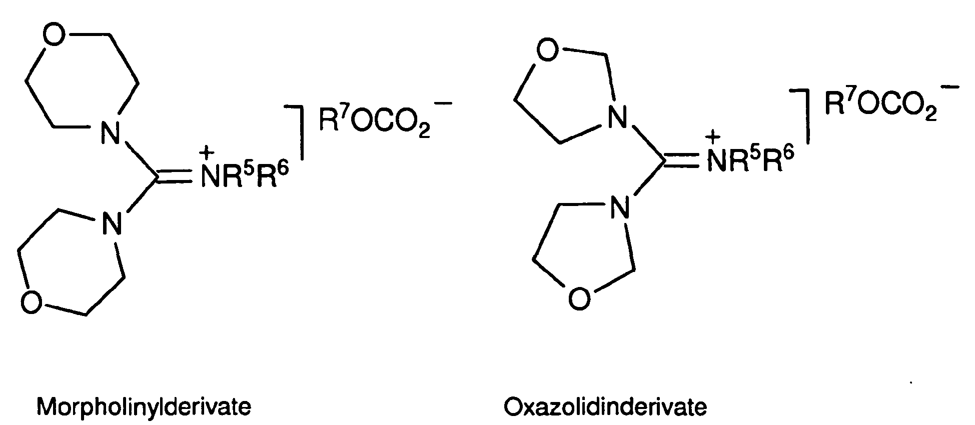 Mit oder ohne zusätzliche organischen Substituenten an den Ringkohlenstoffatomen 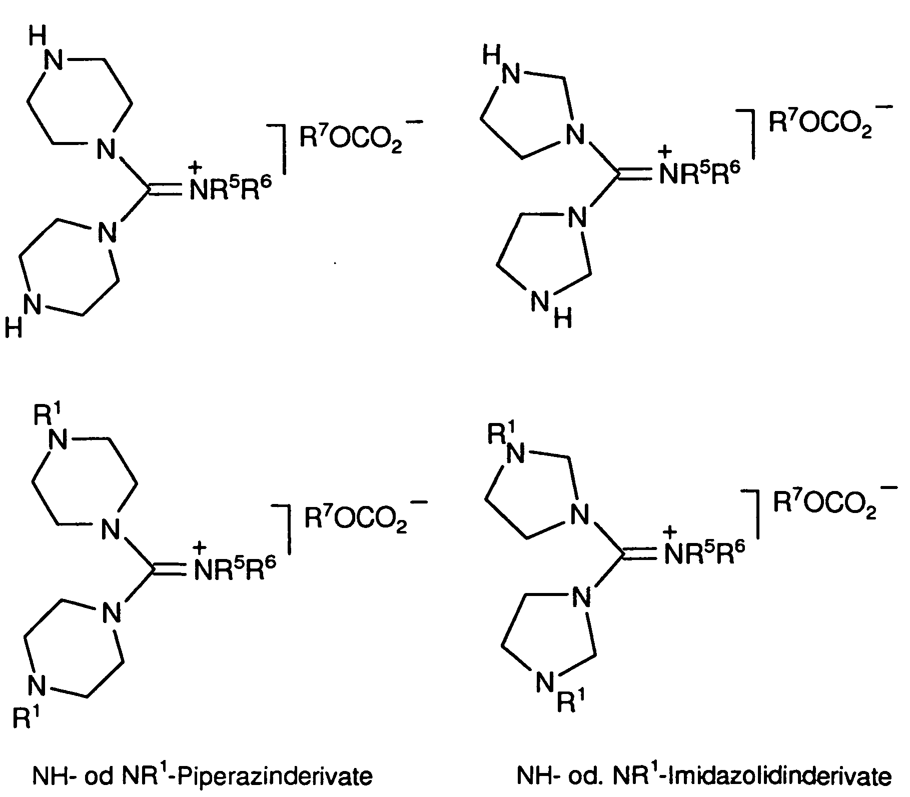 Mit oder ohne zusätzliche organischen Substituenten an den Ringkohlenstoffatomen
4. Wie oben ausgeführt, können die sekundären Amineinheiten -NR¹R² und -NR³R⁴ auch partiell ungesättigt sein: 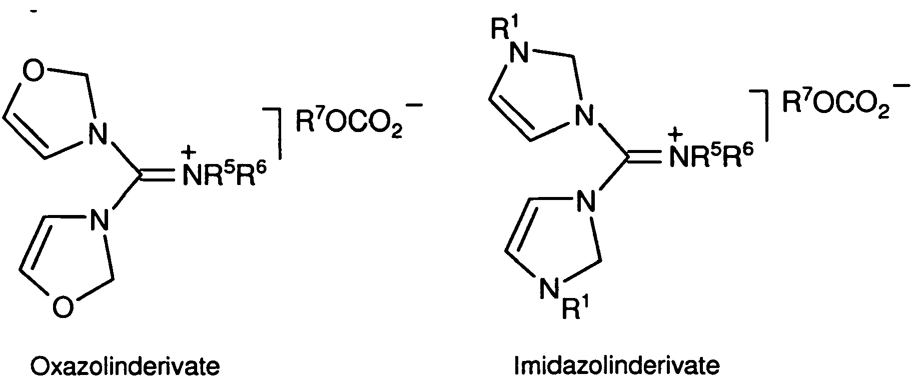 Mit oder ohne zusätzliche organischen Substituenten an den Ringkohlenstoffatomen

In einer weiteren vorteilhaften Ausführungsform handelt es sich um Hexaorganoguanidinium-organocarbonate gemäß Formel (1), bei denen R⁶ eine Methylgruppe und R⁷ eine Methyl-, Benzyl-, Allyl-, Ethyl- oder Phenylgruppe ist. Besonders vorteilhaft sind Hexaorganoguanidinium-organocarbonate gemäß Formel (I), bei denen R⁶ und R⁷ Methylgruppen sind. Bei diesen Verbindungen handelt es sich um (Methyl-pentaorganoguanidinium)-methylcarbonate.
In einer weiteren bevorzugten Ausführungsform handelt es sich bei den beiden Resten R⁶ und R⁷ um zwei Benzylgruppen oder um zwei Allylgruppen. In diesem Fall ist R⁶ im Unterschied zu den anderen vorteilhaften Ausführungsformen natürlich keine Methylgruppe.

In einer weiteren Ausführungsform sind im Guanidiniumkation mindestens zwei der Reste R¹ bis R⁶ voneinander verschieden, so dass es sich um ein unsymmetrisches Guanidiniumkation gemäß obiger Definition handelt. Vorteilhaft ist es hierbei, wenn sich wenigstens die beiden Reste R⁵ und R⁶ voneinander unterscheiden. Ist beispielsweise R⁶ eine Methylgruppe, so handelt es sich also in dieser speziellen Ausführungsform bei mindestens einem der Reste R¹ bis R⁵ nicht um eine Methylgruppe. Analoges gilt für den Fall, dass R⁶ eine Benzyl-, Allyl- oder Ethylgruppe ist.
Besonders vorteilhaft ist es in dieser Ausführungsform, wenn die vier Reste R¹ bis R⁴ identisch sind und vier Methyl-, vier Ethyl- oder vier Butylgruppen darstellen, R⁵ und R⁶ von R¹ bis R⁴ verschieden sind und auch R⁵ ungleich R⁶ ist, wobei R⁶-wie oben aufgeführt - bevorzugt eine Methyl-, Benzyl-, Allyl- oder Ethylgruppe ist. Dem Fachmann ist bekannt, dass unsymmetrisch substituierte Verbindungen niedrigere Schmelzpunkte aufweisen als ähnlich substituierte symmetrische Verbindungen. Erfindungsgemäße unsymmetrisch substituierte Hexaorganoguanidinium-Kationen eignen sich deshalb besonders gut als bei Raumtemperatur flüssige ionische Flüssigkeiten.

Vorteilhaft sind des Weiteren erfindungsgemäße Hexaorganoguanidinium-organocarbonate, bei denen R¹ bis R⁵ unabhängig voneinander lineare oder verzweigte acyclische Alkylgruppen darstellen und R⁶ und R⁷ Methylgruppen sind. Besonders vorteilhaft ist es hierbei, wenn - wie oben für eine andere Ausführungsform beschrieben - mindestens zwei Reste R¹ bis R⁶ voneinander verschieden sind, so dass hier eine der Gruppen R¹ bis R⁵ keine Methylgruppe ist.

Die erfindungsgemäßen Hexaorganoguanidinium-organocarbonate gemäß Formel (I) werden hergestellt, indem ein Pentaorganoguanidin (II) mit einem Diorganocarbonat (III) umgesetzt wird. Anschließend wird nicht umgesetztes Edukt entfernt, beispielsweise bei vermindertem Druck oder durch Extraktion der nichtionischen organischen Ausgangsstoffe mit organischen Lösemitteln wie Alkanen, aromatischen Kohlenwasserstoffen, Ethern und weiteren mit der ionischen Flüssigkeit (IL = ionic liquid) nicht oder wenig mischbaren Lösemitteln.

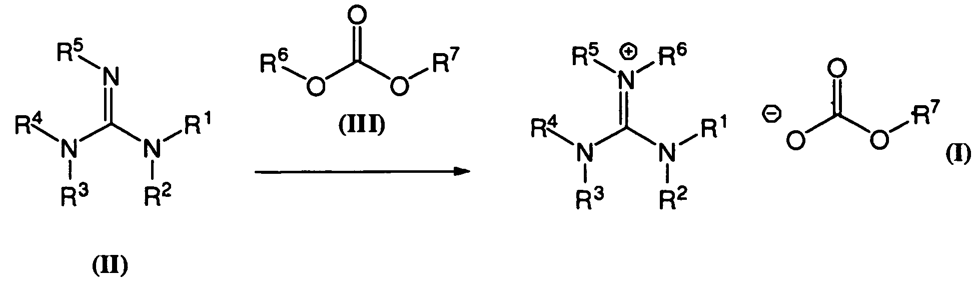

Dabei haben R¹ bis R⁶ die oben genannten Bedeutungen.
R⁷ ist eine Alkylgruppe mit 1 bis 20 Kohlenstoffatomen, eine Benzyl-, Allyl-, Aryl-oder Heteroarylgruppe.
Dabei sind Alkyl-, Benzyl-, Aryl- und Heteroarylgruppe wie oben definiert.

In einer bevorzugten Ausführungsform ist R⁶ eine Methyl-, Benzyl-, Allyl- oder Ethylgruppe. Besonders bevorzugt ist R⁶ eine Methylgruppe. Vorteilhaft einzusetzende Methylorganocarbonate sind beispielsweise das (symmetrische) Dimethylcarbonat (DMC) sowie das (unsymmetrische) Methylphenylcarbonat.

In einer weiteren bevorzugten Ausführungsform handelt es sich um ein symmetrisches Diorganocarbonat, d.h. R⁶ und R⁷sind identisch. Bevorzugte symmetrische Diorganocarbonate sind Dimethylcarbonat (DMC), Diethylcarbonat (DEC), Diallylcarbonat und Dibenzylcarbonat.

Am meisten bevorzugt unter allen erfindungsgemäß einzusetzenden Diorganocarbonaten ist Dimethylcarbonat.

Überraschend wurde gefunden, dass sich Diorganocarbonate zur Einführung organischer Gruppen in Pentaalkylguanidine eignen, und zwar sogar in sterisch anspruchsvoll substituierten. Bislang war bekannt, dass beispielsweise Dimethylcarbonat (DMC) ein sehr schwaches Alkylierungsmittel ist, das nur gegenüber sehr nucleophilen Teilchen wirkt. Daher ist es nicht trivial, statt eines im Stand der Technik bereits bekannten Carboxylats ein Organocarbonat herzustellen. Guanidine sind bekanntermaßen zwar starke Basen, aber keine starken Nucleophile. Aufgrund ihrer extremen Protonenaffinität bzw. starken Basizität in Lösung neigen sie in den bisher bekannten Reaktionen zudem eher zu Eliminierungs- als zu Substitutionsreaktionen. Dieses erwartete Reaktivitätsprinzip wurde überraschend in der vorliegenden Erfindung mit der Generierung einer neuen Stoffklasse der Hexalkylguanidinium-organocarbonate erstmalig überwunden

Beim erfindungsgemäßen Verfahren wird die Gruppe R⁶ auf das Pentaorganoguanidin übertragen. Für die Wanderungsfähigkeit der allen genannten Gruppen R⁶ gilt Methylgruppe > Benzylgruppe > Allylgruppe » Ethylgruppe und höhere Alkylgruppen.

Das erfindungsgemäße Verfahren kann sowohl in der flüssigen als auch in der Gasphase durchgeführt werden.

Sowohl bei der Herstellung in der flüssigen als auch in der Gasphase spielt es keine Rolle, ob zunächst das Pentaorganoguanidin vorgelegt und dann das Diorganocarbonat zugegeben wird oder umgekehrt. Es ist auch möglich, beide Edukte gleichzeitig in einen Reaktionsraum einzubringen.

Wird das erfindungsgemäße Verfahren zur Herstellung von Hexaorganoguanidiniumorganocarbonaten in flüssiger Phase durchgeführt, so ist es vorteilhaft, ein Inertgas zu verwenden, beispielsweise Stickstoff, CO₂ oder Argon. Eindringen von Luftfeuchtigkeit führt zur Hydrolyse des Organocarbonats und Bildung von Hydrogencarbonat und Alkohol R⁷-OH.
Die Reaktionstemperatur beträgt bei Umsetzung in der flüssiger Phase vorzugsweise 20°C bis 250°C, bevorzugt 50°C bis 180°C.

Die beiden Edukte Pentaorganoguanidin (II) und Diorganocarbonat (III) können sowohl bei Umsetzung in der flüssigen als auch in der Gasphase in äquimolaren Verhältnissen oder mit einem Überschuss eines der beiden Edukte (Pentaorganoguanidin oder Diorganocarbonat) eingesetzt werden. Vorteilhaft sind Molverhältnisse von 1 Mol Pentaorganoguanidin zu 0,1 bis 10 Mol Diorganocarbonat.

In einer bevorzugten Ausführungsform wird das Diorganocarbonat in äquimolaren Mengen oder im Überschuss zum Pentaorganoguanidin eingesetzt. Bevorzugt sind in diesem Fall Molverhältnisse von 1 Mol Pentaorganoguanidin zu 1,0 bis 6,0 Mol Diorganocarbonat.

Falls Pentaorganoguanidin und Diorganocarbonat nicht in äquimolaren Mengen eingesetzt wurden, kann der im Überschuss eingesetzte Reaktand nach beendeter Reaktion durch Anlegen von Vakuum aus dem Produkt entfernt und anschließend beispielsweise in einem neuen Reaktionszyklus wieder eingesetzt werden.

Wird die Umsetzung der beiden Edukte in flüssiger Phase durchgeführt, so kann die Reaktion in Gegenwart oder in Abwesenheit von Lösungsmitteln durchgeführt werden. Geeignete Lösungsmittel sind beispielsweise, aber nicht erschöpfend:
- Alkohole, z.B. Methanol, Ethanol, n-Propanol, iso-Propanol, Cyclohexanol,
- Nitrile wie z.B. Acetonitril, Propionitril,
- aromatische Kohlenwasserstoffe wie z.B. Benzol, Toluol, Xylole,
- aliphatische Kohlenwasserstoffe und deren Gemische wie z.B. Ligroin, Petrolether, Dekan, Hexan, Methylcyclohexan
- Ether wie Methyl-tert.-butylether, Diethylether, Diisopropylether, Di-n-butylether, Tetrahydrofuran, Dioxan, 1,2-Dimethoxyethan.
- Chlorkohlenwasserstoffe wie Dichlormethan, Chloroform, Chlorbenzol, 1,1,1-Trichlorethan.
Bei Umsetzung in flüssiger Phase ist es - wie oben schon allgemein beschrieben - vorteilhaft, das Diorganocarbonat in äquimolaren Mengen oder im Überschuss zum Pentaorganoguanidin einzusetzen.
Nach beendeter Reaktion wird nicht umgesetztes Edukt bei vermindertem Druck entfernt. Optional kann das Reaktionsprodukt mit einem inerten Lösungsmittel gewaschen werden, beispielsweise mit einem Alkan wie n-Pentan oder n-Hexan.

In einer weiteren Ausführungsform werden die flüchtigen Edukte in der Gasphase bei Temperaturen zwischen 30 °C und 300 °C bei einem Gesamtdruck von von 10⁻¹ mbar bis 10 bar umgesetzt. Dem Fachmann ist bekannt, welche Edukte er wie stark erwärmen muss, um sie in die Gasphase zu überführen. Optional können das Erwärmen der Edukte und die Gasphasenreaktion bei vermindertem Druck durchgeführt werden. Bei der Gasphasenreaktion scheidet sich das nicht flüchtige Produkt als Flüssigkeit ab und kann auf diese Weise leicht von den Edukten getrennt werden. Optional kann das Produkt wie oben beschrieben mit einem inerten Lösungsmittel gewaschen werden.

Die unter Formel (I) und dem obigen Verfahren beschriebenen Hexaorganoguanidinium-organocarbonate sind ionische Flüssigkeiten mit einem Organocarbonatanion.

Das obige Verfahren ist besonders vorteilhaft, weil erstmals Pentaorganoguanidine in einer zu 100 % atomökonomischen Direktsynthese umgesetzt werden, sofern Pentaorganoguanidin und Diorganocarbonat in äquimolarem Verhältnis eingesetzt werden. Unter Atomökonomie wird der prozentuale Anteil der in einer chemischen Reaktion von den Edukten in die Produkte überführten Atome verstanden.
Das Problem der Selektivität, das beispielsweise bei Imidazol-basierten Umsetzungen mit Dimethylcarbonat wegen der Ring-Carboxylierung auftritt, stellt sich bei der vorliegenden Erfindung nicht. Des Weiteren sind beim erfindungsgemäßen Verfahren Druckreaktionsgefäße oder Autoklaven nicht zwingend notwendig, so lange die Reaktion in der Gasphase bei Drücken kleiner oder gleich dem Normdruck von etwa 1 bar oder in flüssiger Phase durchgeführt wird. Nur bei Durchführung der Reaktion in der Gasphase bei Drücken über 1 bar sind entsprechend druckstabile Reaktionsgefäße vonnöten. Dagegen sind bei den Imidazol-basierten Umsetzungen in jedem Fall aufwändige Druckreaktionen in Methanol als Lösungsmittel erforderlich.

Die erfindungsgemäßen Hexaorganoguanidinium-organocarbonate können für die Herstellung von ionischen Flüssigkeiten verwendet werden. Die Organocarbonatanionen von Verbindungen gemäß Formel (I) sind basisch und lassen sich mit beliebigen Säuren HX und sauren Salzen decarboxylieren, wodurch beliebige Anionen in die ionischen Flüssigkeiten eingeführt werden können.
Die Reaktion ist eine spezielle Anion-Metathese-Reaktion, bei der sich das Organocarbonatanion in Gegenwart des Protons der Säure HX zu CO₂ und einem Alkohol zersetzt. Daher erhält man bei dieser Metathese kein Gleichgewicht wie beim klassischen Anionenaustausch, sondern es kommt - vor allem durch die Freisetzung von CO₂ - zu einer Gleichgewichtsverschiebung, weshalb die erfindungsgemäße Metathesereaktion quantitativ verläuft. Das gebildete CO₂ kann aufgefangen und beispielsweise für die Herstellung von neuem Diorganocarbonat verwendet werden, und der gebildete Alkohol lässt sich auf vielerlei Weise verwenden, beispielsweise als Lösungsmittel oder ebenfalls als Ausgansstoff für die Herstellung eines Diorganocarbonats. Damit entstehen bei dieser Reaktion keine toxischen oder besonders schwierig zu entsorgenden Nebenprodukte, und die entstehenden Nebenprodukte lassen sich ökonomisch nutzen.

Die Acidität der Säure HX oder des sauren Salzes [NH₄]X wird so gewählt, dass das Organocarbonatanion gemäß Formel (I) - vorzugsweise ein Methylcarbonatanion - zum entsprechenden Alkohol, beispielsweise Methanol, und Kohlendioxid zersetzt wird. Auch Wasser kann in der erfindungsgemäßen Metathese als Säure HX verwendet werden. In Gegenwart von Wasser bildet sich aus dem Organocarbonatanion durch Hydrolyse zunächst ein Hydrogencarbonatanion, so dass die die Metathesereaktion im Ergebnis kein Hydroxid, sondern dessen Abfangprodukt mit Kohlendioxid ergibt. Die Eignung einer Säure HX oder des sauren Salzes [NH₄]X für die Metathese lässt sich leicht ermitteln, da geeignete Säuren und Salze aus den erfindungsgemäßen Hexaorganoguanidinium-organocarbonaten Kohlendioxid freisetzen.

Die Metathese von erfindungsgemäßen Hexaorganoguanidinen ist beispielhaft für die Umsetzung mit einer Säure HX gezeigt:

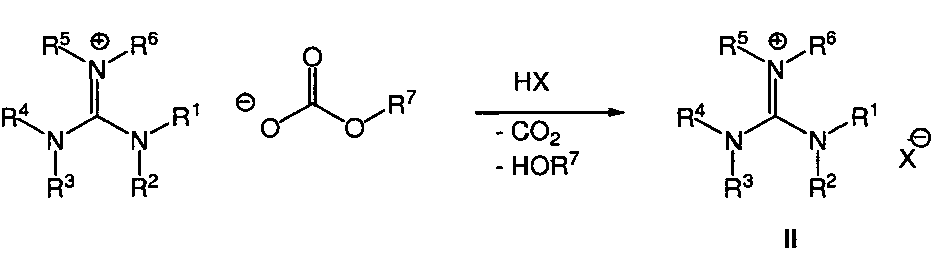

Beim Anion X kann es sich, abgesehen von einem Organocarbonatanion, um ein beliebiges Anion handeln, dessen Basizität geringer ist als die des Organocarbonations. Sollte das Anion X basischer sein als das Organocarbonat, so kann das Gleichgewicht durch Abfangreaktion des in situ gebildeten Anions X mit einem Elektrophil verschoben werden. Typisches Beispiel hierfür ist die Abfangreaktion von X = OH durch CO₂.

Das Anion X- der Säure oder des Salzes tritt an die Stelle des Organocarbonatanions. Geeignete Säuren sind Protonensäuren, geeignete saure Salze sind solche, welche protischen Wasserstoff im Kation enthalten, etwa NH₄⁺, Et₃NH⁺, H₃O⁺, R^{a}₃PH⁺ oder welche protischen Wasserstoff im Anion enthalten, etwa HSO₄⁻, H₂PO₄⁻, R^{a}PO₃H⁻.

Die Umsetzung kann bei Normaldruck durchgeführt werden. Ein Überdruck ist nicht notwendig, aber auch nicht hinderlich.

Erhöhte Temperaturen können hilfreich sein; bevorzugt sind Temperaturen von 20 bis 200 °C, insbesondere 90 bis 150 °C.

Die Säure oder das Salz, welche das Anion X⁻ enthält, kann in beliebigen Mengen, je nach gewünschtem Umsatz, eingesetzt werden. Vorzugsweise wird die Säure bzw. das Salz im Überschuss oder in äquimolaren Mengen eingesetzt. Insbesondere kann die gewünschte bzw. benötigte Menge Säure oder Salz vorab durch Titration bestimmt werden.

Das Anion X- wird vorzugsweise so gewählt, dass das Hexaorganoguanidiniumsalz der Formel II einen Schmelzpunkt (bei 1 bar) kleiner 200 °C, insbesondere kleiner 100 °C, besonders bevorzugt kleiner 50 °C und ganz besonders bevorzugt kleiner 30 °C hat, also eine unter Normalbedingungen flüssige ionische Flüssigkeit ist.

In Betracht kommen insbesondere Verbindungen mit einer Carboxylatgruppe (kurz Carboxylate). Bevorzugte Carboxylate sind die Anionen von Alkancarbonsäuren, insbesondere von C1- bis C4-Alkancarbonsäuren, vorzugsweise das Acetat-Anion.

Geeignete Anionen können weiter aus den nachstehenden Gruppen ausgewählt werden (Aufgeführte mehrwertige Anionen sind die Gegenanionen einer der Wertigkeit entsprechenden Zahl von Hexaorganoguanidinium-Kationen, R^{a}, R^{b}, R^{c} und R^{d} stehen unabhängig voneinander für einen organischen Rest mit vorzugsweise 1 bis 20 Kohlenstoffatomen, besonders bevorzugt für einen Aryl-, Heteroaryl-, Allyl-, Benzyl-, Vinyl- oder Alkylrest und ganz besonders bevorzugt für einen aliphatischen Rest, insbesondere eine C1- bis C10-Alkylgruppe. Optional können die Reste R^{a}, R^{b}, R^{c} und R^{d} ganz oder teilweise fluoriert sein.):
- der Gruppe der Halogenide, Pseudohalogenide, Halogenometallate und weiteren halogenhaltigen Verbindungen der Formeln
   F⁻, HF₂⁻, Cl⁻, Br⁻, I⁻, BF₄⁻, PF₆⁻, AICl₄⁻, Al₂Cl₇⁻, Al₃Cl₁₀⁻, AlBr₄⁻, FeCl₄⁻, BCl₄⁻, SbF₆⁻, AsF₆⁻, ZnCl₃⁻, SnCl₃⁻, CᵤCl₂⁻, CF₃SO₃⁻, (CF₃SO₃)₂N⁻, CF₃COO⁻, CN⁻, SCN⁻, OCN⁻, NO₂⁻, NO₃⁻, N₃⁻;
- der Gruppe der Carboxylate der allgemeinen Formel R^{a}-CO₂⁻, C₂O₄²⁻, F₃CCO₂⁻, PhCO₂⁻ (Benzoat), Polyacrylate;
- der Gruppe der Sulfate, Sulfite und Sulfonate der allgemeinen Formeln SO₄²⁻, HSO₄⁻, SO₃²⁻, HSO₃⁻, R^{a}OSO₃⁻, R^{a}SO₃⁻, Polystryolsulfonate;
- der Gruppe der Phosphate der allgemeinen Formeln PO₄³⁻ , HPO₄²⁻, H₂PO₄⁻, R^{a}PO₄⁻, R^{a}R^{b}PO₄⁻;
- der Gruppe der Phosphonate und Phosphinate der allgemeinen Formeln R^{a}HPO₃⁻_{,} R^{a}R^{b}PO₂⁻_{,} R^{a}R^{b}PO₃⁻;
- der Gruppe der Phosphite der allgemeinen Formeln PO₃³⁻, HPO₃²-, H₂PO₃⁻, R^{a}PO₃²⁻, R^{a}HPO₃⁻, R^{a}R^{b}PO₃⁻;
- der Gruppe der Phosphonite und Phosphinite der allgemeinen Formeln R^{a}R^{b}PO₂⁻, R^{a}HPO₂⁻, R^{a}R^{b}PO⁻, R^{a}HPO⁻;
- der Gruppe der Borate der allgemeinen Formeln BO₃³⁻, HBO₃²⁻, H₂BO₃⁻, R^{a}R^{b}BO₃⁻, R^{a}HBO₃⁻, R^{a}BO₃²⁻, B(OR^{a})(OR^{b})(OR^{c}),(OR^{d})⁻, B(HSO₄)⁻, B(R^{a}SO₄)⁻,
- der Gruppe der Boronate der allgemeinen Formeln R^{a}BO₂⁻_{,} R^{a}R^{b}BO⁻,
- der Gruppe der Silikate und Kieselsäureester der allgemeinen Formeln SiO₄⁴⁻ , HSiO₄³⁻ , H₂SiO₄²⁻ , H₃SiO₄⁻, R^{a}SiO₄³⁻ , R^{a}R^{b}SiO₄²⁻ R^{a}R^{b}R^{c}SiO₄⁻, HR^{a}SiO₄²⁻, H₂R^{a}SiO₄⁻, HR^{a}R^{b}SiO₄⁻, partiell deprotoniertes Silica;
- der Gruppe der Alkyl- bzw. Arylsilanolat-Salze der allgemeinen Formeln R^{a}SiO₃³⁻, R^{a}R^{b}SiO₂²⁻, R^{a}R^{b}R^{c}SiO⁻, R^{a}R^{b}R^{c}SiO₃⁻, R^{a}R^{b}R^{c}SiO₂⁻, R^{a}R^{b}SiO₃²⁻,
- der Gruppe der Carbonsäureimide, Bis(sulfonylimide) und Sulfonylimide der allgemeinen Formeln
   N(COOR^{a})₂⁻, N(COOR^{a})(COOR^{b})⁻, N(SO₂R^{a})₂⁻ N(SO₂R^{a})(SO₂R^{b})⁻, NR^{a}(SO₂R^{b})⁻
- der Gruppe der Polycyanoanionen N(CN)₂⁻, C(CN)₃⁻, B(CN)₄⁻,

Bevorzugte Anionen sind die obigen Carboxylate, insbesondere Acetat, Trifluoracetat, Rhodanid (SCN⁻), Alkylsulfate, Alkylsulfonate wie Methansulfonat und Ethansulfonat, Arylsulfonate wie Tosylat und Bis(sulfonylimide) wie N(SO₂CF₃)₂⁻, ferner BF₄⁻, PF₆⁻ sowie B(CN)₄⁻ und N(CN)₂⁻.

Die Säuren der Anionen X⁻ können als solche direkt eingesetzt werden, z.B. HN₃, HCl HSO₃^{a}, (CF₃SO₃)₂NH, CF₃SO₂OH, CH₃COOH. Alternativ können sie beispielsweise aus Ammoniumsalzen freigesetzt werden, z.B. aus NH₄N₃, NH₄SCN, NH₄(SO₃R^{a}). Allgemein eignen sich hierfür H-funktionelle Ammoniumsalze, d.h. NH₄⁺-Salze, aber auch Ammoniumsalze primärer, sekundärer und tertiärer Amine der allgemeinen Formeln NR^{a}H₃⁺, NR^{a}R^{b}H₂⁺ und NR^{a}R^{b}R^{c}H⁺. Des Weiteren können die Säuren auch aus protolytischen Säuregeneratoren freigesetzt werden, beispielsweise aus Triorganosilanen R^{a}₃Si-X und Alkohol, worin R^{a} bevorzugt eine Methylgruppe ist, der Alkohol bevorzugt MeOH und X beispielsweise für N₃, SCN, R^{a}SO₃ oder CF₃SO₃ steht.

Optional kann die Anionmetathese-Reaktion mit HX in Gegenwart einer Lewissäure wie beispielsweise CuCl₂, FeCl₃, AlCl₃ oder BF₃ stattfinden.

Nachfolgend sind beispielhaft, aber nicht erschöpfend einige mögliche Metathese-Reaktionen genannt:

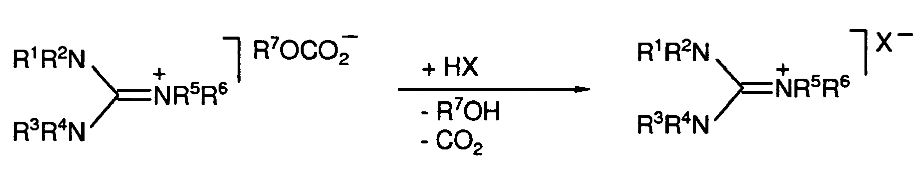

Hierbei kann HX zum Beispiel CH₃COOH, CF₃COOH oder HBF₄-Etherat sein.

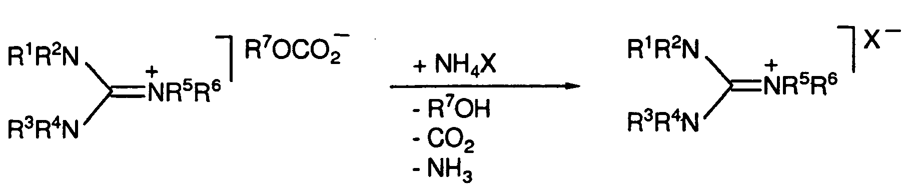

NH₄X ist beispielsweise NH₄SCN.

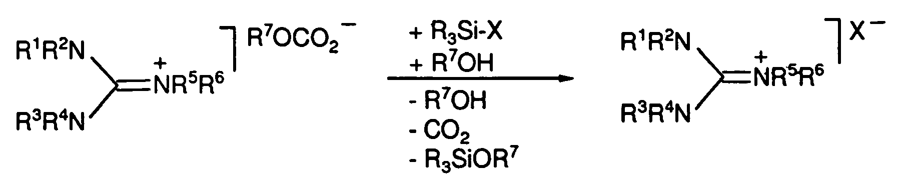

R₃SiX ist beispielsweise Trimethylsilylazid, R⁷OH ist beispielsweise MeOH.

Die Anion-Metathese-Reaktionen können ohne zusätzliche Lösungsmittel durchgeführt werden. In diesem Fall ist das eingesetzte Hexaorganoguanidiniumorganocarbonat Lösungsmittel und Reaktand zugleich. Alternativ können diese Reaktionen in inerten dipolar-aprotischen Lösungsmitteln wie Acetonitril, DMF, Chlorbenzol, Ethern, Ketonen, Carbonsäureestern oder aber in protolytisch aktiven Lösungsmitteln wie Alkoholen (z.B. Methanol, Ethanol), Carbonsäuren (z.B. Ameisensäure, Essigsäure, Propionsäure) oder Wasser durchgeführt werden.

Die erfindungsgemäßen Hexaorganoguanidinium-organocarbonate und die daraus über Metathese zugänglichen weiteren Salze von Hexaorganoguanidinium-Kationen sind thermisch stabile ionische Flüssigkeiten. Vor allem solche Hexaorganoguanidinium-organocarbonate und weitere Salze von Hexaorganoguanidinium-Kationen, deren Schmelzpunkt kleiner 200 °C, insbesondere bei Raumtemperatur flüssige Substanzen, besitzen vielfältige Verwendungsmöglichkeiten. Sie gewinnen zunehmende Bedeutung beispielsweise als technische Lösungsmittel oder Betriebsmittel, z.B. zum Auflösen von Cellulose oder als Adsorptionsmittel in Adsorptionswärmepumpen. Des Weiteren können sie als Schmiermittel, Filmmaterialien für die Immobilisierung von Katalysatoren auf festen Trägern (SILP), als Elektrolyte für die Akkutechnologie oder für Anwendungen in farbstoffsensibilisierten Solarzellen verwendet werden.

### Ausführungsbeispiele

Nachfolgend wird eine vereinfachte Nomenklatur der Alkylguanidine und Guanidiniumkationen verwendet.

Ein Guanidin ist durch die Substituenten an den drei Stickstoffatomen *N, N'* und *N"* gekennzeichnet.
Diese werden angehängt an das Kürzel "Gua" angegeben, wobei zunächst die Substituenten an *N,* dann jene an *N',* schließlich jene an N" genannt werden.
Die Substituenten an ein und demselben Stickstoffatom werden durch Kommata, solche an unterschiedlichen Stickstoffatomen durch Bindestriche getrennt. Guanidiniumionen haben somit immer sechs Bezeichner und werden in eckigen Klammern geschrieben, Guanidine haben dagegen fünf Bezeichner und werden in runden Klammern geschrieben.
Eine Alkylgruppe wird dabei durch eine Zahl entsprechend der Zahl der C-Atome des Substituenten gekennzeichnet, z. B.:

| | |
|---|---|
| Hydrogen = 0 | Ethyl = 2 |
| n-Octyl = 8 | 2-Ethylhexyl (Isooctyl) = i8 |
| Phenyl = Ph | Cyclohexyl = c6 |

### Anmerkungen zur Priorität:

Die Reihenfolge der anzugebenden Substituenten richtet sich nach der Reihenfolge der Stickstoffatome des Guanidins bzw. Guanidiniumions im IUPAC-konformen Namen der Verbindung. Somit steht der Substituent des (formal) doppelt gebundenen N-Atoms im Guanidin immer in der Mitte, die Reihenfolge aller übrigen N-Atome (*N* und *N"* bei Guanidinen, *N, N'* und *N"* bei Guanidiniumionen) sowie die Reihenfolge der Substituenten an jedem doppelt substituierten N-Atom richtet sich nach den allgemeinen IUPAC-Prioritätsregeln.

Im Rahmen der vorliegenden Erfindung wurden die folgenden Guanidine als Startmaterialien verwendet:
(Gua-1,1-1-1,1), (Gua-2,2-4-2,2), (Gua-2,2-i8-2,2), (Gua-4,4-4-4,4), (Gua-4,4-i8-4,4)

### Ausführungsbeispiel 1:

### Herstellung von N,N,N;N;N';N"-Hexamethylguanidinium-methylcarbonat [Gua-1,1-1,1-1,1](MeOCO₂)

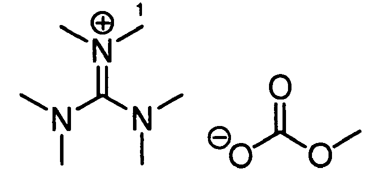

Ein Gemisch aus 761 mg (5.89 mmol, 1.00 eq) *N,N,N',N",N"-*Pentamethylguanidin und 5.35 g (5.0 mL, 59.3 mmol, 10.1 eq) Dimethylcarbonat wurde in einen 25 mL-Schlenkkolben mit Teflonventil gegeben, entgast und unter Inertgasatmosphäre für 3 d auf 120 °C erhitzt. Das Reaktionsgemisch wurde im Feinvakuum bei Raumtemperatur von überschüssigem Dimethylcarbonat befreit, zweimal mit je 20 mL Pentan gewaschen und im Feinvakuum getrocknet.
**Ausbeute:** 1.20 g (93%) farbloses, kristallines Pulver.

**Tₘ**: 125-128 °C.
**¹H-NMR** (300 MHz, CD₃CN): δ = 3.13 (s, 18H, C1-*H*), 3.31 (s, 3H, OC*H*₃) ppm.
**¹³C-NMR** (75 MHz, CD₃CN): δ = 51.8 (O*C*H₃), 56.1 (t, ¹ *J*_{NC} = 4.1 Hz, *C*1 ) ppm.
**IR** (ATR, in Substanz): ν̃ = 2937 (w, v(C-H)_{ges.}), 1655 (vs, ν(C=O)), 1498 (m), 1277 (vs, ν(C-O)), 1069 (s, ν(C-O)), 953 (s), 869 (s), 825 (m), 581 (m), 461 (m) cm⁻¹.

### Ausführungsbeispiel 2:

### Herstellung von N-n-Butyl-N,N,N",N"-tetraethyl-N-methylguanidinium-methylcarbonat [Gua-4,1-2,2-2,2](MeOCO₂)

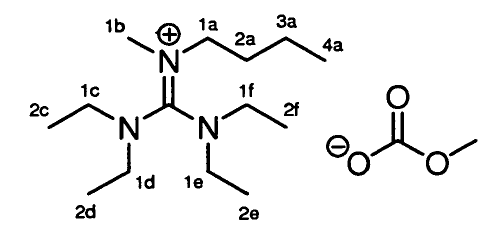

Ein Gemisch aus 1.88 g (8.27 mmol, 1.00 eq) *N'-n-Butyl-N,N,N",N"-*tetraethylguanidin und 4.49 g (4.2 mL, 49.84 mmol, 6.03 eq) Dimethylcarbonat wurde in einen 25 mL-Schlenkkolben mit Teflonventil gegeben, entgast und unter Inertgasatmosphäre für 3 d auf 120 °C erhitzt. Das Reaktionsgemisch wurde im Feinvakuum bei Raumtemperatur von überschüssigem Dimethylcarbonat befreit, zweimal mit je 20 mL Pentan gewaschen und im Feinvakuum getrocknet.
**Ausbeute:** 2.53 g (97%) hellgelbes Öl.
**¹H-NMR** (300 MHz, CD₃CN): δ = 0.91 (t, 3H, ³*J*_{HH} = 7.3 Hz, C4a-*H*), 1.08-1.17 (m, 12H, C2c-f-*H*), 1.21-1.36 (m, 2H, C3a-*H*), 1.43-1.72 (m, 2H, C2a-*H*), 2.85 (s, 3H, C1b-*H*), 3.06-3.34 (m, 10H, C1 a,c-f-*H*), 3.28 (s, 3H, OC*H*₃) ppm.
**¹³C-NMR** (75 MHz, CD₃CN): δ = 12.9, 13.1, 13.2, 13.4 (C2c-f), 14.0 (C4a), 20.7 (C3a), 30.0 (C2a), 38.6 (*C*1b), 44.2, 44.46, 44.61, 44.9 (*C*1c-f), 51.8 (O*C*H₃), 53.4 (C1a), 157.6 (CO₃), 164.7 (CN₃) ppm.
**ESI-MS** (MeCN): pos.: *mlz* (%) = 242 (100) [Kation]⁺.
**ESI-HRMS** (MeCN):C₁₄H₃₂N₃ [Kation]⁺ ber. 242.2591, gef. 242.2590.
**IR** (ATR, in Substanz): ν̃ = 2962 (m, ν(C-H)_{ges.}), 2933 (m, ν(C-H)_{ges.}), 2874 (m, ν (C-H)_{ges.}), 1676 (s, v(C=O)), 1538 (s, ν(C=N)), 1439 (m), 1268 (vs, ν(C-O)), 1064 (s, ν(C-O)), 838 (m) cm⁻¹.
- **CHN**: C₁₆H₃₅N₃O₃ (317.47 g/mol): ber. C 60.53, H 11.11, N 13.24%;
gef. C 60.54, H 11.02, N 13.53%.

### Ausführungsbeispiel 3:

**Herstellung von *N*-(2-Ethylhexyl)-*N'*,*N'*,*N"*,*N"*-tetraethyl-*N*-methylguanidinium-methylcarbonat [Gua-i8,1-2,2-2,2](MeOCO₂)**

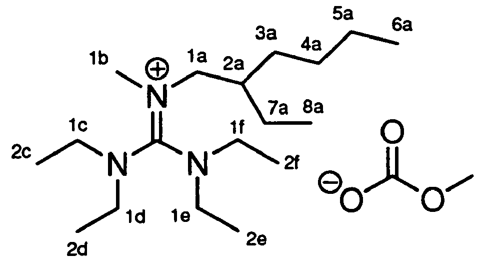

Ein Gemisch aus 2.26 g (10.01 mmol, 1.00 eq) N'-(2-Ethylhexyl)-N,N,N",N"-tetraethylguanidin und 4.49 g (4.2 mL, 49.84 mmol, 4.98 eq) Dimethylcarbonat wurde in einen 25 mL-Schlenkkolben mit Teflonventil gegeben, entgast und unter Inertgasatmosphäre für 3 d auf 120 °C erhitzt. Das Reaktionsgemisch wurde im Feinvakuum bei Raumtemperatur von überschüssigem Dimethylcarbonat befreit, zweimal mit je 20 mL Pentan gewaschen und im Feinvakuum getrocknet.
**Ausbeute:** 2.81 g (95%) hellgelbes Öl.
**¹H-NMR** (300 MHz, CD₃CN): δ = 0.82-0.94 (m, 6H, C6a-H und C8a-*H*), 1.02-1.49 (m, 20H, C1 c-f-H, C3a-H bis C5a-H, C7a-*H*), 1.65-1.75 (m, 1 H, C2a-*H*), 2.87 (s, 3H, C1b-*H*), 2.90-3.38 (m, 10H, C1 a,c-f-*H*), 3.29 (s, 3H, OC*H*₃) ppm.
**¹³C-NMR** (75 MHz, CD₃CN): δ = 10.3, 11.4 (C8a), 12.8, 12.9, 13.0, 13.1, 13.41, 13.43 (C2c-f), 14.3, 14.4 (C6a), 23.7, 23.9, 24.7, 24.8 (C4a und C5a), 28.8, 29.6 (C3a), 31.2, 31.8 (C7a), 37.3, 37.8 (C2a), 39.4 (*C*1b), 44.1, 44.3, 44.56, 44.58, 44.9 (C1 c-f), 51.8 (O*C*H₃), 57.4, 57.6 (C1 a), 157.6 (*C*O₃), 165.2 (*C*N₃) ppm.
Durch das Stereozentrum des 2-Ethylhexyl-Substituenten in Verbindung mit der schraubenartig verdrillten Umgebung des zentralen trigonalen Kohlenstoffatoms werden zwei diastereomere Kationen erzeugt, die das ¹³C-NMR-Spektrum in Form eines doppelten Signalsatzes zeigt.
**ESI-MS** (MeCN): pos.: *mlz* (%) = 298 (100) [Kation]⁺.
**ESI-HRMS** (MeCN):C₁₈H₄₀N₃ [Kation]⁺ ber. 298.3217, gef. 298.3214.
**IR** (ATR, in Substanz): ν̃ = 2960 (m, ν(C-H)_{ges.}), 2930 (m, ν(C-H)_{ges.}), 2874 (m, ν (C-H)_{ges.}), 1676 (s, ν(C=O)), 1537 (s, ν(C=N)), 1439 (m), 1268 (vs, ν(C-O)), 1065 (s, ν(C-O)), 839 (m) cm⁻¹.

### Ausführungsbeispiel 4:

### Herstellung von N,N,N',N',N"-Penta-n-butyl-N"-methylguanidinium-methylcarbonat [Gua-4,4-4,4-4,1](MeOCO₂)

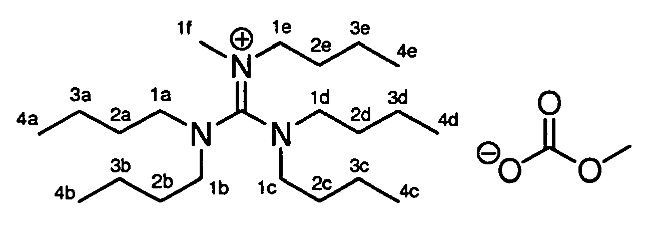

Ein Gemisch aus 5.35 g (15.75 mmol, 1.00 eq) *N,N,N',N",N"-Penta-n-*butylguanidin und 7.09 g (6.7 mL, 78.75 mmol, 5.00 eq) Dimethylcarbonat wurde in einen 250 mL-Schlenkkolben mit Teflonventil gegeben, entgast und unter Inertgasatmosphäre für 3 d auf 120 °C erhitzt. Das nunmehr zweiphasige Reaktionsgemisch wurde im Feinvakuum bei Raumtemperatur von überschüssigem Dimethylcarbonat befreit, zweimal mit je 15 mL Pentan gewaschen und im Feinvakuum getrocknet.
**Ausbeute:** 6.44 g (95%) hellgelbes Öl.
**¹H-NMR** (300 MHz, CD₃CN): δ = 0.91, 0.92 (2xt, 15H, ³*J*_{HH} = 7.2, 7.3 Hz, C4a-e-*H*), 1.20-1.49 (m, 15H, C3a-e-H, C2a-e-*H*^{a}), 1.56-1.75 (m, 5H, C2a-e-*H*^{b}), 2.86 (s, 3H, C1f-*H*), 2.95-3.26 (m, 10H, C1a-e-*H*^{a} und -*H*^{b}), 3.29 (s, 3H, OC*H*₃) ppm.
**¹³C-NMR** (75 MHz, CD₃CN): δ = 14.0 (C4a-e), 20.7 (C3e), 20.8 (C3a-d), 30.07, 30.09, 30.2, 30.4, 30.5 (C2a-e), 38.7 (*C*1f), 49.8, 50.1, 50.2, 50.6 (*C*1a-d), 51.8 (OCH₃), 53.6 (C1 e), 157.6 (*C*Oₛ), 165.0 (*C*N₃) ppm.
- **ESI-MS** (MeCN):: pos.: *mlz* (%) = 355 (100) [Kation]⁺;
neg.: *mlz* (%) = 75 (100) [Anion]⁻.
- **ESI-HRMS** (MeCN):: C₂₂H₄₈N₃ [Kation]⁺ ber. 354.3843, gef. 354.3838;
C₂H₃O₃ [Anion]⁻ ber. 75.0088, gef. 75.0090.
**IR** (ATR, in Substanz): ν̃ = 2956 (m, ν(C-H)_{ges.}), 2931 (m, ν(C-H)_{ges.}), 2872 (m, ν (C-H)_{ges.}), 1676 (vs, ν(C=O)), 1534 (s, ν(C=N)), 1457 (m), 1433 (m), 1269 (vs, ν(C-O)), 1066 (s, ν(C-O)), 841 (s) cm⁻¹.

### Ausführungsbeispiel 5:

### Herstellung von N',N',N",N"-Tetra-n-butyl-N-(2-ethylhexyl)-N-methylguanidinium-methylcarbonat [Gua-i8,1-4,4-4,4](MeOCO₂)

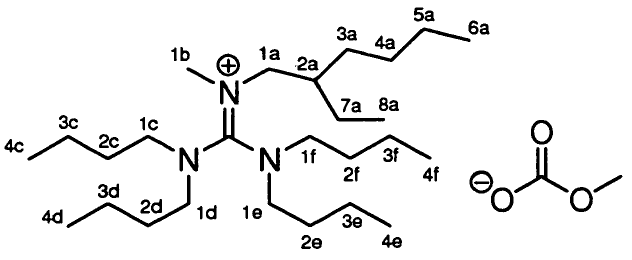

Ein Gemisch aus 1.75 g (4.41 mmol, 1.00 eq) *N,N,N",N"*-Tetra-*n*-butyl-*N'*-(2-ethylhexyl)-guanidin und 5.35 g (5.0 mL, 59.3 mmol, 13.45 eq) Dimethylcarbonat wurde in einen 50 mL-Schlenkkolben mit Teflonventil gegeben, entgast und unter Inertgasatmosphäre für 3 d auf 120 °C erhitzt. Das Reaktionsgemisch wurde im Feinvakuum bei Raumtemperatur von überschüssigem Dimethylcarbonat befreit, zweimal mit je 20 mL Pentan gewaschen und im Feinvakuum getrocknet.
**Ausbeute:** 1.93 g (90%) blassgelbliches Öl.
**¹H-NMR** (300 MHz, CD₃CN): δ = 0.82-0.95 (m, 18H, C4c-f-*H*, C6a-*H* und C8a-*H*), 1.02-1.49 (m, 20H, C3c-f-*H*, C2c-f-*H*^{a}, C3a-*H* bis C5a-*H*, C7a-*H*), 1.50-1.79 (m, 5H, C2a-*H*, C2c-f-*H*^{b}), 2.87 (s, 3H, C1b-*H*), 2.90-3.31 (m, 10H, C1a,c-f-*H*), 3.29 (s, 3H, OC*H*₃) ppm.
**¹³C-NMR** (75 MHz, CD₃CN): δ = 10.6, 11.2 (*C*8a), 13.95, 13.98, 14.00, 14.05 (*C*4c-f), 14.3, 14.4 (*C*6a), 20.7, 20.80, 20.83, 20.92 (*C*3c-f), 23.7, 24.0, 24.7, 24.8 (*C*4a und *C*5a), 29.0, 29.5 (*C*3a), 30.22, 30.29, 30.35, 30.39, 30.46, 30.49 (*C*2c-f), 31.1, 32.0 (*C*7a), 37.4, 37.7 (*C*2a), 39.3, 39.4 (*C*1b), 49.9, 50.00, 50.09, 50.10, 50.17, 50.22, 50.7 (*C*1c-f), 51.8 (O*C*H₃), 57.7, 57.8 (*C*1a), 157.6 (*C*O₃), 165.4 (*C*N₃) ppm.

Durch das Stereozentrum des 2-Ethylhexyl-Substituenten in Verbindung mit der schraubenartig verdrillten Umgebung des zentralen trigonalen Kohlenstoffatoms werden zwei diastereomere Kationen erzeugt, die das ¹³C-NMR-Spektrum in Form eines doppelten Signalsatzes zeigt.

**IR** (ATR, in Substanz): ν̃ = 2956 (m, ν(C-H)_{ges.}), 2930 (m, ν(C-H)_{ges.}), 2872 (m, ν (C-H)_{ges.}), 1677 (vs, ν(C=O)), 1532 (s, ν(C=N)), 1457 (m), 1434 (m), 1269 (vs, ν(C-O)), 1066 (s, ν(C-O)), 882 (m) cm⁻¹.
- **CHN**: C₂₈H₅₉N₃O₃ (485.79 g/mol): ber. C 69.23, H 12.24, N 8.65%;
gef. C 68.77, H 11.93, N 9.36%.

### Ausführungsbeispiel 6:

### Herstellung von N,N,N',N',N"-Penta-n-butyl-N"-methylguanidinium-acetat [Gua-4,4-4,4-4,1 ](OAc)

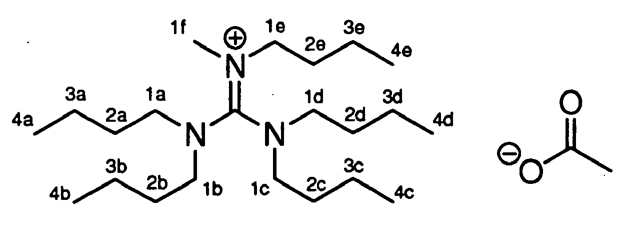

In einem 30 mL-Schlenkkolben wurden 433 mg (1.01 mmol, 1.00 eq) *N*,*N*,*N'*,*N'*,*N"*-Penta-*n*-butyl-*N"*-methylguanidinium-methylcarbonat in 5 mL Methanol gelöst und mit 61 mg (58 µL, 1.01 mmol, 1.00 eq) Essigsäure versetzt. Das Gemisch wurde unter Inertgasatmosphäre für 12 h bei 50 °C gerührt. Nach dem Abkühlen wurde das Methanol im Vakuum entfernt und das Produkt für 2 h bei 80 °C im Hochvakuum getrocknet.
**Ausbeute:** 343 mg (82%) leicht bräunliches Öl.
**¹H-NMR** (300 MHz, CD₃CN): δ = 0.91, 0.92 (2xt, 15H, ³*J*_{HH} = 7.2, 7.3 Hz, C4a-e-*H*), 1.98-1.50 (m, 15H, C3a-e-H, C2a-e-*H*^{a}), 1.54-1.75 (m, 5H, C2a-e-*H*^{b}), 1.75 (s, 3H, C(=O)C*H*₃), 2.86 (s, 3H, C1f-*H*), 2.93-3.28 (m, 10H, C1a-e-*H*^{a} und -*H*^{b}) ppm.
**¹³C-NMR** (75 MHz, CD₃CN): δ = 14.0 (*C*4a-e), 20.7 (*C*3e), 20.8 (*C*3a-d), 24.3 (C(=O) *C*_{H}3), 30.08, 30.10, 30.2, 30.3, 30.5 (*C*2a-e), 38.7 (*C*1f), 49.8, 50.16, 50.22, 50.6 (*C*1a-d), 53.6 (*C*1e), 165.0 (*C*N₃), 174.9 (*C*(=0)CH₃) ppm.
**ESI-MS** (MeCN): pos.: *mlz* (%) = 355 (100) [Kation]⁺.
**ESI-HRMS** (MeCN):C₂₂H₄₈N₃ [Kation]⁺ ber. 354.3843, gef. 354.3840.
**IR** (ATR, in Substanz): ν̃ = 2957 (m, ν(C-H)_{ges.}), 2931 (m, ν(C-H)_{ges.}), 2872 (m, ν (C-H)_{ges.}), 1589 (s, νₐₛ(CO₂⁻)), 1534 (vs, ν(C=N)), 1457 (m), 1418 (m), 1363 (s, ν ₛ(CO₂‾)), 1314 (w), 737 (w) cm⁻¹.

### Ausführungsbeispiel 7:

### Herstellung von N,N,N',N',N"-Penta-n-butyl-N"-methylguanidinium-trifluoracetat [Gua-4,4-4,4-4,1][TFA]

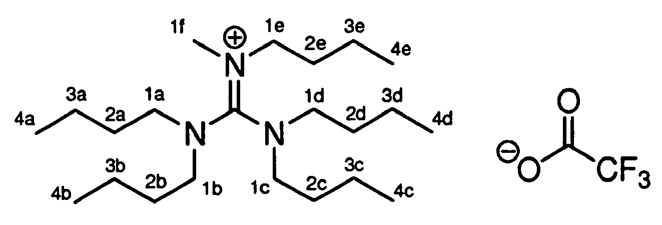

In einem 30 mL-Schlenkkolben wurden 432 mg (1.00 mmol, 1.00 eq) *N*,*N*,*N'*,*N'*,*N"*-Penta-*n*-butyl-*N"*-methylguanidinium-methylcarbonat in 5 mL Methanol gelöst und mit 150 mg (0.1 mL, 1.30 mmol, 1.30 eq) Trifluoressigsäure versetzt. Das Gemisch wurde unter Inertgasatmosphäre für 12 h bei 50 °C gerührt. Nach dem Abkühlen wurde das Methanol im Vakuum entfernt und das Produkt im Feinvakuum getrocknet.
**Ausbeute:** 439 mg (94%) schwach gelbliches Öl.
**¹H-NMR** (300 MHz, CD₃CN): δ = 0.91, 0.92 (2xt, 15H, ³*J*_{HH} = 7.2, 7.3 Hz, C4a-e-*H*), 1.20-1.49 (m, 15H, C3a-e-H, C2a-e-*H*^{a}), 1.56-1.75 (m, 5H, C2a-e-*H*^{b}), 2.86 (s, 3H, C1f-*H*), 2.95-3.29 (m, 10H, C1a-e-*H*^{a} und -*H*^{b}) ppm.
**¹³C-NMR** (75 MHz, CD₃CN): δ = 14.0 (*C*4a-e), 20.7 (*C*3e), 20.8 (*C*3a-d), 30.07, 30.10, 30.2, 30.3, 30.5 (*C*2a-e), 38.7 (*C*1f), 49.9, 50.16, 50.22, 50.6 (*C*1a-d), 53.6 (*C*1 e), 118.7 (q, ¹*J*_{FC} = 299 Hz, *C*F₃), 159.0 (q, ²*J*_{FC} = 32 Hz, C=O), 165.0 (*C*N₃) ppm.
**¹⁹F-NMR** (376 MHz, CD₃CN): δ = -73.6 (CF₃, ¹*J*_{FC} = 297 Hz) ppm.
- **ESI-MS** (MeCN):: pos.: *mlz* (%) = 355 (100) [Kation]⁺;
neg.: m/z (%) = 113 (100) [Anion]⁻.
**ESI-HRMS** (MeCN):C₂₂H₄₈N₃ [Kation]⁺ ber. 354.3843, gef. 354.3841.
**IR** (ATR, in Substanz): ν̃ = 2959 (m, ν(C-H)_{ges.}), 2933 (m, ν(C-H)_{ges.}), 2874 (w, ν (C-H)_{ges.}), 1690 (vs, ν(C=O)), 1535 (s, ν(C=N)), 1458 (w), 1194 (s, ν(C-O)), 1149 (s, ν(C-F)), 1106 (vs, ν(C-F)), 811 (m, 8(C-F)), 796 (m, 8(C-F)), 714 (m, <5(C-F)) cm⁻¹.

### Ausführungsbeispiel 8:

### Herstellung von N,N,N',N',N"-Penta-n-butyl-N"-methylguanidinium-thiocyanat [Gua-4,4-4,4-4,1 ](SCN)

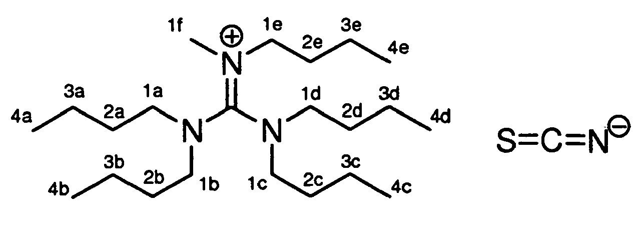

In einem 30 mL-Schlenkkolben wurden 425 mg (0.99 mmol, 1.00 eq) *N*,*N*,*N'*,*N'*,*N"*-Penta-*n*-butyl-*N"*-methylguanidinium-methylcarbonat in 5 mL Methanol gelöst und mit 78 mg (1.02 mmol, 1.03 eq) Ammoniumthiocyanat versetzt. Das Gemisch wurde unter Inertgasatmosphäre für 12 h bei 50 °C gerührt. Nach dem Abkühlen wurde das Methanol im Vakuum entfernt, das Produkt mit 10 mL Diethylether digeriert und im Feinvakuum getrocknet.
**Ausbeute:** 327 mg (80%) hell orangefarbenes Öl.
**¹H-NMR** (300 MHz, CD₃CN): δ = 0.91, 0.92 (2xt, 15H, ³*J*_{HH} = 7.2, 7.3 Hz, C4a-e-*H*), 1.21-1.52 (m, 15H, C3a-e-H, C2a-e-*H*^{a}), 1.56-1.77 (m, 5H, C2a-e-*H*^{b}), 2.88 (s, 3H, C1f-*H*), 2.95-3.29 (m, 10H, C1a-e-*H*^{a} und -*H*^{b}) ppm.
**¹³C-NMR** (75 MHz, CD₃CN): δ = 14.0 (*C*4a-e), 20.7 (*C*3e), 20.8 (*C*3a-d), 30.07, 30.10, 30.2, 30.3, 30.5 (*C*2a-e), 38.7 (*C*1f), 49.8, 50.17, 50.22, 50.6 (*C*1a-d), 53.6 (*C*1e), 131.3 (S*C*N), 165.0 (*C*N₃) ppm.
- **ESI-MS** (MeCN): pos.:: *mlz* (%) = 355 (100) [Kation]⁺;
neg.: *mlz* (%) = 58 (100) [Anion]⁻.
- **ESI-HRMS** (MeCN):: C₂₂H₄₈N₃ [Kation]⁺ ber. 354.3843, gef. 354.3839;
CNS [Anion]⁻ ber. 57.9757, gef. 57.9757.
**IR** (ATR, in Substanz): ν̃ = 2957 (m, ν(C-H)_{ges.}), 2931 (m, ν(C-H)_{ges.}), 2871 (m, ν(C-H)_{ges.}), 2051 (s, ν(C=N)), 1534 (vs, ν(C=N)), 1457 (m), 1436 (m), 1417 (m), 1378 (w), 1313 (w), 891 (w), 734 (w) cm⁻¹.

### Ausführungsbeispiel 9:

### Herstellung von N,N,N',N',N"-Penta-n-butyl-N"-methylguanidinium-azid [Gua-4,4-4,4-4,1 ](N₃)

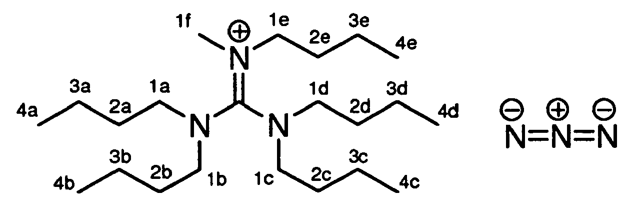

In einem 30 mL-Schlenkkolben wurden 883 mg (2.06 mmol, 1.00 eq) *N*,*N*,*N'*,*N'*,*N"*-Penta-*n*-butyl-*N"*-methylguanidinium-methylcarbonat in 5 mL Methanol gelöst und mit 350 mg (0.4 mL, 3.05 mmol, 1.48 eq) Trimethylsilylazid versetzt. Das Gemisch wurde unter Inertgasatmosphäre für 12 h bei 50 °C gerührt. Nach dem Abkühlen wurde das Methanol im Vakuum entfernt und das Produkt im Feinvakuum getrocknet.
**Ausbeute:** 809 mg (99%) hellgelbes Öl.
**¹H-NMR** (300 MHz, CD₃CN): δ = 0.91, 0.92 (2xt, 15H, ³*J*_{HH} = 7.2, 7.3 Hz, C4a-e-*H*), 1.87-1.49 (m, 15H, C3a-e-H, C2a-e-*H*^{a}), 1.52-1.74 (m, 5H, C2a-e-*H*^{b}), 2.87 (s, 3H, C1f-*H*), 2.95-3.26 (m, 10H, C1a-e-*H*^{a} und -*H*^{b}) ppm.
**¹³C-NMR** (75 MHz, CD₃CN): δ = 14.0 (C4a-e), 20.7 (C3e), 20.8 (C3a-d), 30.10, 30.13, 30.3, 30.4, 30.5 (C2a-e), 38.7 (*C*1f), 49.9, 50.18, 50.24, 50.6 (*C*1a-d), 53.6 (C1e), 165.0 (*C*N₃) ppm.
**ESI-MS** (MeCN): pos.: *mlz* (%) = 355 (100) [Kation]⁺.
**ESI-HRMS** (MeCN):C₂₂H₄₈N₃ [Kation]⁺ ber. 354.3848, gef. 354.3841.

**IR** (ATR, in Substanz): ν̃ = 2956 (m, ν(C-H)_{ges.}), 2931 (m, ν(C-H)_{ges.}), 2871 (m, ν(C-H)_{ges.}), 1990 (vs, ν(N=N=N)), 1534 (s, ν(C=N)), 1457 (m), 1418 (m), 1377 (w), 1314 (w), 890 (w), 738 (w) cm⁻¹.

### Ausführungsbeispiel 10:

### Herstellung von N,N,N',N',N"-Penta-n-butyl-N"-methylguanidinium-tetrafluoroborat [Gua-4,4-4,4-4,1](BF4)

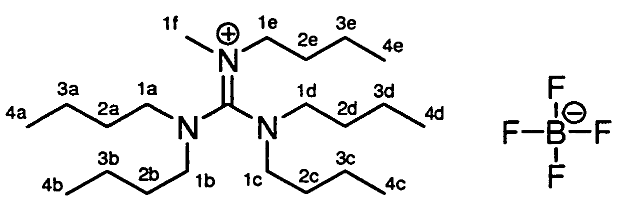

In einem 30 mL-Schlenkkolben wurden 436 mg (1.01 mmol, 1.00 eq) *N*,*N*,*N'*,*N'*,*N"*-Penta-*n*-butyl-*N"*-methylguanidinium-methylcarbonat in 5 mL Methanol gelöst und mit 0.2 mL 50%-iger Tetrafluoroborsäure in Diethylether (entspricht 1.52 mmol, 1.50 eq) versetzt. Das Gemisch wurde unter Inertgasatmosphäre für 12 h bei 50 °C gerührt. Nach dem Abkühlen wurde das Methanol im Vakuum entfernt und das Produkt im Feinvakuum getrocknet.

**Ausbeute:** 441 mg (99%) farbloses Öl.
**¹H-NMR** (300 MHz, CD₃CN): δ = 0.92, 0.93 (2xt, 15H, ³*J*_{HH} = 7.2, 7.3 Hz, C4a-e-*H*), 1.20-1.49 (m, 15H, C3a-e-H, C2a-e-*H*^{a}), 1.52-1.74 (m, 5H, C2a-e-*H*^{b}), 2.86 (s, 3H, C1f-*H*), 2.95-3.28 (m, 10H, C1a-e-*H*^{a} und -*H*^{b}) ppm.
**¹³C-NMR** (75 MHz, CD₃CN): δ = 14.0 (C4a-e), 20.7 (C3e), 20.8 (C3a-d), 30.09, 30.13, 30.2, 30.4, 30.5 (C2a-e), 38.7 (*C*1f), 49.9, 50.2, 50.3, 50.7 (*C*1a-d), 53.7 (C1e), 165.0 (*C*N3) ppm.
**¹⁹F-NMR** (376 MHz, CD₃CN): δ = 150.5 ppm.

**ESI-MS** (MeCN):
pos.: *mlz* (%) = 355 (100) [Kation]⁺;
neg.: *mlz* (%) = 87 (100) [Anion]⁻.

**ESI-HRMS** (MeCN):
C₂₂H₄₈N₃ [Kation]⁺ ber. 354.3843, gef. 354.3840;
BF₄ [Anion]⁻ ber. 87.0035, gef. 87.0035.

**IR** (ATR, in Substanz): ν̃ = 2959 (w, ν(C-H)_{ges.}), 2933 (w, ν(C-H)_{ges.}), 2873 (w, ν(C-H)_{ges.}), 1536 (m, ν(C=N)), 1459 (w), 1090 (m), 1047 (vs, ν(B-F)), 1033 (s), 887 (w), 519 (w) cm⁻¹.

## Patentansprüche

1. Eine Verbindung der Formel (I) 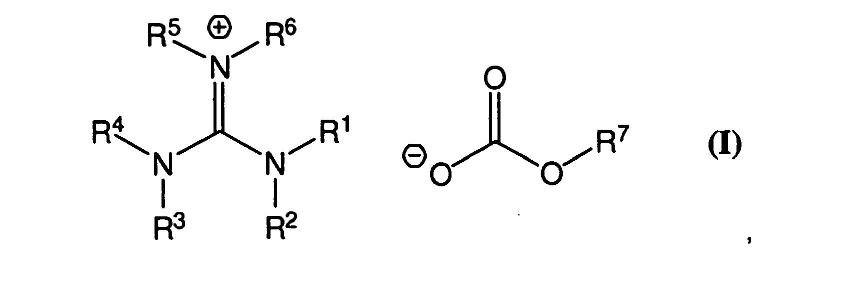 worin
R¹, R², R³, R⁴ und R⁵ unabängig voneinander für eine lineare oder verzweigte acyclische oder cyclische Alkylgruppe mit 1 bis 20 Kohlenstoffatomen, eine Allyl- oder Vinylgruppe, eine Benzylgruppe, eine Aryl- oder Heteroarylgruppe stehen
oder
worin das Paar R¹ und R² sowie das Paar R³ und R⁴ unabhängig voneinander eine lineare oder verzweigte Alkylengruppe mit 2 bis 8 Kohlenstoffatomen darstellen, die jeweils mit einem Stickstoffatom des Guanidingerüstes eine cyclische sekundäre Aminogruppe -NR¹R² bzw. -NR³R⁴ bildet,
oder
worin das Paar R² und R³ eine lineare oder verzweigte Alkylengruppe mit 2 bis 6 Kohlenstoffatomen darstellt, die mit dem zentralen Kohlenstoffatom und zwei Stickstoffatomen des Guanidingerüstes eine cyclische Gruppe ∼R²-N-C-N-R³∼ bildet,
wobei in allen genannten Alkylengruppen ein -CH₂-Einheit optional durch O, NH oder NR¹ ersetzt sein kann, worin R¹ wie oben definiert ist, und worin cyclische Guppen -NR¹R² und -NR³R⁴ partiell ungesättigt sein können,
wobei im Falle des Vorliegens cyclischer Gruppen -NR¹R², NR³R⁴ bzw. R²-N-C-N-R³ diejenigen Reste R¹ bis R⁴, welche nicht an den cyclischen Gruppen beteiligt sind, wie oben definiert sind,
und worin
R⁶ eine lineare oder verzweigte acyclische Alkylgruppe mit 1 bis 20 Kohlenstoffatomen, eine Benzyl- oder eine Allylgruppe ist,
und R⁷ eine lineare oder verzweigte acyclische oder cyclische Alkylgruppe mit 1 bis 20 Kohlenstoffatomen, eine Benzyl- oder eine Allylgruppe, eine Aryl- oder eine Heteroarylgruppe ist.

2. Verbindung gemäß Anspruch 1, **dadurch gekennzeichnet, dass** R⁶ eine Methylgruppe ist.

3. Verbindung gemäß einem der Ansprüche 1 und 2, **dadurch gekennzeichnet, dass** die Reste R¹ bis R⁵ lineare oder verzweigte acyclische Alkylgruppen sind.

4. Verbindung gemäß einem der Ansprüche 1 und 2, **dadurch gekennzeichnet, dass** es sich um Hexaorganoguanidinium-organocarbonate mit einer cyclischen sekundären Aminogruppe ∼R²-N-C-N-R³∼ handelt.

5. Verbindung gemäß einem der Ansprüche 1 und 2, **dadurch gekennzeichnet, dass** es sich um Hexaorganoguanidinium-organocarbonate mit cyclischen sekundären Aminogruppen -NR¹R² und -NR³R⁴ handelt.

6. Verbindung gemäß einem der Ansprüche 1 und 3 bis 5, **dadurch gekennzeichnet, dass** R⁶ und R⁷ zwei Methyl-, zwei Benzyl- oder zwei Allylgruppen sind.

7. Verbindung gemäß einem der Ansprüche 1 bis 6, **dadurch gekennzeichnet, dass** mindestens zwei der Reste R¹ bis R⁶ voneinander verschieden sind.

8. Verfahren zur Herstellung von Verbindungen gemäß einem der Ansprüche 1
bis 7, **dadurch gekennzeichnet, dass** ein Pentaorgano-guanidin (II) 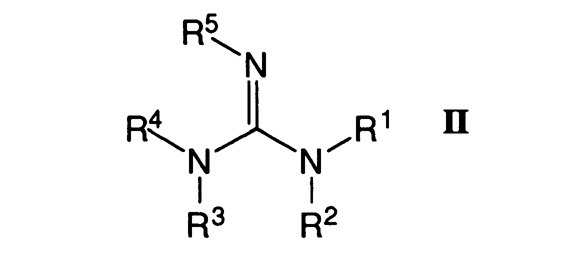 mit einem Diorganocarbonat (III) 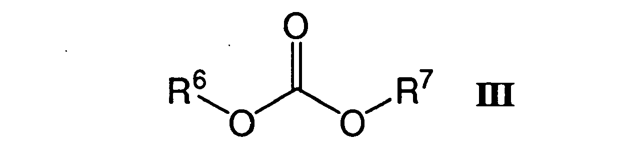 umgesetzt und anschließend nicht umgesetztes Edukt entfernt wird,
wobei
R¹, R², R³, R⁴, R⁵ und R⁶ die oben angegeben Bedeutungen haben und
R⁷ eine Alkylgruppe mit 1 bis 20 Kohlenstoffatomen, eine Benzyl-, Allyl-, Aryl-, oder Heteroarylgruppe ist.

9. Verfahren gemäß Anspruch 8, **dadurch gekennzeichnet, dass** das Diorganocarbonat (III) Dimethylcarbonat, Dibenzylcarbonat, Diallylcarbonat oder Diethylcarbonat ist.

10. Verfahren gemäß einem der Ansprüche 8 und 9, **dadurch gekennzeichnet, dass** die Umsetzung in flüssiger Phase bei einer Reaktionstemperatur von 20 °C bis 300 °C durchgeführt wird.

11. Verfahren gemäß einem der Ansprüche 8 und 9, **dadurch gekennzeichnet, dass** die Umsetzung in der Gasphase bei einer Reaktionstemperatur von 30 °C und 300 °C und einem Gesamtdruck von 10⁻¹ mbar bis 10 bar durchgeführt wird.

12. Verfahren gemäß einem der Ansprüche 8 bis 11, **dadurch gekennzeichnet,**
**dass** die Reaktanden in einem Molverhältnis von 1 Mol Pentaorganoguanidin (II) zu 0,1 bis 10,0 Mol Diorganocarbonat (III) eingesetzt werden.

13. Verwendung von Hexaorganoguanidinium-organocarbonaten gemäß einem der Ansprüche 1 bis 7 in einer Anionen-Metathese-Reaktion, worin die Hexaorganoguanidinium-organocarbonate mit einer Säure HX, direkt oder in situ generiert, oder einem sauren Salz decarboxyliert werden, und das Anion X⁻ der Säure oder des sauren Salzes an die Stelle des Organocarbonats tritt.

## Claims

1. A compound according to formula (I) 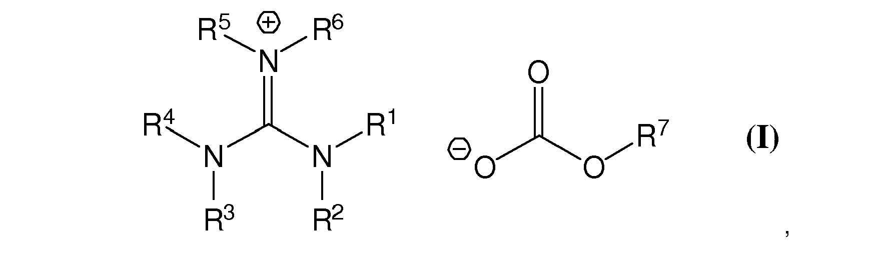 wherein
R¹, R², R³, R⁴ and R⁵ stand independently of one another for a linear or branched acyclic or cyclic alkyl group with 1 to 20 carbon atoms, an allyl or vinyl group, a benzyl group, an aryl or heteroaryl group.
or
wherein the pair R¹ and R² and the pair R³ and R⁴ represent, independently of one another, a linear or branched alkylene group with 2 to 8 carbon atoms, each of which forms, with one nitrogen atom of the guanidine skeleton, a cyclic secondary amino group -NR¹R² and -NR³R⁴, respectively,
or
wherein the pair R² and R³ represents a linear or branched alkylene group with 2 to 6 carbon atoms, which forms a cyclic group ∼R²-N-C-N-R³∼ with the central carbon atom and two nitrogen atoms of the guanidine skeleton, wherein in all mentioned alkylene groups, one -CH₂ unit can be optionally replaced by O, NH or NR¹, wherein R¹ is defined as above, and
wherein cyclic groups -NR¹R² und -NR³R⁴ can be partially unsaturated, wherein, in the case of the existence of cyclic groups -NR¹R², NR³R⁴ or R²-N-C-N-R³, respectively, those residues R¹ to R⁴, which are not involved in the cyclic groups, are defined as above,
and wherein
R⁶ is a linear or branched acyclic alkyl group with 1 to 20 carbon atoms, a benzyl or an allyl group,
and R⁷ is a linear or branched acyclic or cyclic alkyl group with 1 to 20 carbon atoms, a benzyl or an allyl group, an aryl or a heteroaryl group.

2. Compound according to claim 1, wherein R⁶ is a methyl group.

3. Compound according to one of claims 1 and 2, wherein the residues R¹ to R⁵ are linear or branched acyclic alkyl groups.

4. Compound according to one of claims 1 and 2, wherein it belongs to hexaorgano guanidinium organocarbonates with a cyclic secondary amino group ∼R²-N-C-N-R³∼.

5. Compound according to one of claims 1 and 2, wherein it belongs to hexaorgano guanidinium organocarbonates with cyclic secondary amino groups -NR¹R² and -NR³R⁴_{.}

6. Compound according to one of claims 1 and 3 to 5, wherein R⁶ and R⁷ are two methyl, two benzyl or two allyl groups.

7. Compound according to one of claims 1 to 6, wherein at least two of the residues R¹ to R⁶ are different to each other.

8. Method for the production of compounds according to one of the claims 1 to 7,
wherein a pentaorgano guanidine (II) is reacted with 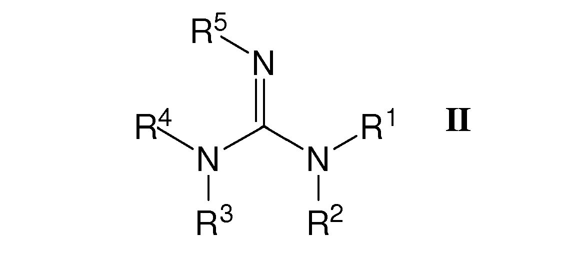 a diorgano carbonate (III) 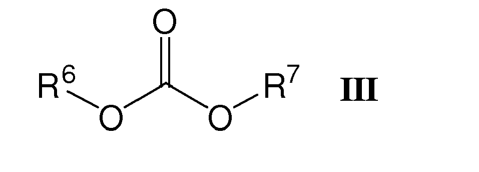 and unreacted starting material is subsequently removed,
wherein
R¹, R², R³, R⁴, R⁵ and R⁶ have the meanings indicated above and
R⁷ is an alkyl group with 1 to 20 carbon atoms, a benzyl, allyl, aryl or heteroaryl group.

9. Method according to claim 8, wherein the diorgano carbonate (III) is dimethyl carbonate, dibenzyl carbonate, diallyl carbonate or diethyl carbonate.

10. Method according to one of the claims 8 and 9, wherein the reaction is carried out in liquid phase at a reaction temperature of 20°C to 300°C.

11. Method according to one of the claims 8 and 9, wherein the reaction is carried out in gas phase at a reaction temperature of 30°C to 300°C and a total pressure of 10⁻¹ mbar to 10 bar.

12. Method according to one of claims 8 to 11, wherein the reactants are used at a molar ratio of 1 mol pentaorgano guanidine (II) to 0.1 to 10.0 mol diorgano carbonate (III).

13. Use of hexaorgano guanidinium organocarbonates according to one of claims 1 to 7 in an anion metathesis reaction, wherein the hexaorgano guanidinium organocarbonates are decarboxylated with an acid HX, which is generated directly or in situ, or an acid salt, and the anion X⁻ of the acid or the acid salt takes the place of the organo carbonate.

## Revendications

1. Un composé de la formule (I) 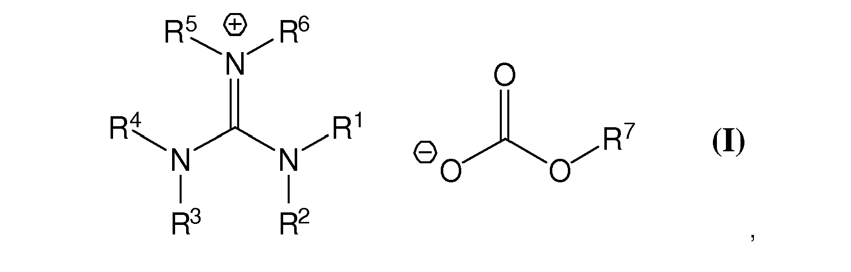 où
R¹, R², R³, R⁴ et R⁵ représentent indépendamment les uns des autres un groupe alkyle acyclique linéaire ou ramifié ou cyclique linéaire ou ramifié avec 1 à 20 atomes de carbone, un groupe allyle ou vinyle, un groupe benzyle, un groupe aryle ou hétéroaryle
ou
où la paire R¹ et R² ainsi que la paire R³ et R⁴ représentent indépendamment l'une de l'autre un groupe alkylène linéaire ou ramifié avec 2 à 8 atomes de carbone, chacun formant un groupe amino cyclique secondaire -NR¹R² ou -NR³R⁴ avec un atome de carbone de la structure de guanidine,
ou
où la paire R² et R³ représente un groupe alkylène linéaire ou ramifié avec 2 à 6 atomes de carbone, qui forme un groupe cyclique ∼R²-N-C-N-R³∼ avec l'atome de carbone central et deux atomes d'azote de la structure de guanidine,
où une unité -CH₂ peut être optionnellement remplacé par O, NH ou NR¹ dans tous les groupes alkylènes mentionnés, où R¹ est tel que défini ci-dessus, et où des groupes cycliques -NR¹R² et -NR³R⁴ peuvent être partiellement insaturés,
où dans le cas de la présence de groupes cycliques -NR¹R², NR³R⁴ ou R²-N-C-N-R³ des résidus R¹ à R⁴ qui ne sont pas associés dans les groupes cycliques, tels que définis ci-dessus,
et où
R⁶ est un groupe alkyle acyclique linéaire ou ramifié avec 1 à 20 atomes de carbone, un groupe benzyle ou un groupe allyle,
et R⁷ est un groupe alkyle acyclique ou cyclique linéaire ou ramifié ou un groupe alkyle cyclique linéaire ou ramifié avec 1 à 20 atomes de carbone, un groupe benzyle ou un groupe allyle, un groupe aryle ou hétéroaryle.

2. Composé selon la revendication 1, **caractérisé en ce que** R⁶ est un groupe méthyle.

3. Composé selon l'une des revendications 1 et 2, **caractérisé en ce que** les résidus R¹ à R⁵ sont des groupes alkyles acycliques linéaires ou ramifiés.

4. Composé selon l'une des revendications 1 et 2, **caractérisé en ce qu'**il s'agit d'organocarbonate d'hexaorganoguanidinium avec un groupe amino cyclique secondaire ∼R²-N-C-N-R³∼.

5. Composé selon l'une des revendications 1 et 2, **caractérisé en ce qu'**il s'agit d'organocarbonate d'hexaorganoguanidinium avec des groupes amino cycliques secondaires -NR¹R² und -NR³R⁴_{.}

6. Composé selon l'une des revendications 1 et 3 à 5, **caractérisé en ce que** R⁶ et R⁷ sont deux groupes méthyles, benzyles ou allyles.

7. Composé selon l'une des revendications 1 et 6, **caractérisé en ce qu'**au moins deux des résidus R¹ à R⁶ sont différents l'un de l'autre.

8. Procédé pour la préparation de composés selon l'une des revendications 1 à 7,
**caractérisé en ce qu'**une guanidine pentaorgano (II) est mise en réaction 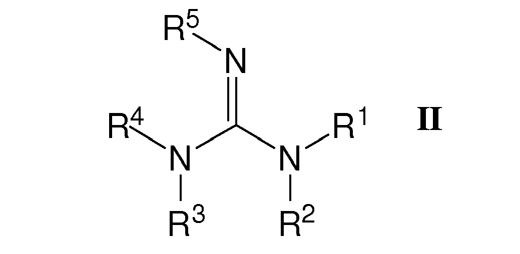 avec un diorganocarbonate (III) 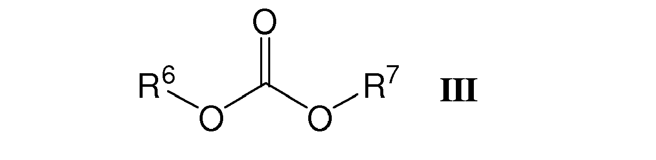 et **en ce qu'**ensuite le produit de départ n'ayant pas réagi est éliminé,
où
R¹, R², R³, R⁴, R⁵ et R⁶ ont les significations indiquées ci-dessus et
R⁷ est un groupe alkyle avec 1 à 20 atomes de carbone, un groupe benzyle, allyle, aryle ou hétéroaryle.

9. Procédé selon la revendication 8, **caractérisé en ce que** le diorganocarbonate (III) est un carbonate de diméthyle, un carbonate de dibenzyle, un carbonate de diallyle ou un carbonate de diéthyle.

10. Procédé selon l'une des revendications 8 et 9, **caractérisé en ce que** la réaction en phase liquide est effectuée à une température de réaction de 20°C à 300°C.

11. Procédé selon l'une des revendications 8 et 9, **caractérisé en ce que** la réaction en phase gazeuse est effectuée à une température de réaction de 30°C et 300°C et sous une pression totale de 10⁻¹ mbar à 10 bar.

12. Procédé selon l'une des revendications 8 à 11, **caractérisé en ce que** les réactifs sont utilisés dans un rapport molaire de 1 mole de guanidine pentaorgano (II) à 0,1 à 10,0 mole de diorganocarbonate (III).

13. Utilisation d'organocarbonates d'hexaorganoguanidinium selon l'une des revendications 1 à 7 dans une réaction anions-métathèse, où les organocarbonates d'hexaorganoguanidinium sont décarboxylés avec un acide HX, généré directement ou in situ, ou avec un sel acide ok et l'anion X⁻ de l'acide ou du sel acide se substitue à l'organocarbonate.
